# EUROPEAN PATENT SPECIFICATION

(11) **EP 2 729 812 B1**
(45) Date of publication and mention of the grant of the patent: **03.05.2017**
(21) Application number: 12733128.8
(22) Date of filing: 06.07.2012
(51) Int. Cl.: G01N 33/574, C07K 14/705, A61K 38/17

(54) **PLA2R1 AS ANTI-TUMORAL COMPOUND AND AS BIOMARKER FOR THE DETECTION OF CANCER**
PLA2R1 ALS ANTITUMORVERBINDUNG UND ALS BIOMARKER ZUR ERKENNUNG VON KREBS
PLA2R1 EN TANT QUE COMPOSÉ ANTITUMORAL ET EN TANT QUE BIOMARQUEUR POUR LA DÉTECTION DU CANCER

(30) Priority: 08.07.2011 EP 11305885; 08.07.2011 US 201161505887 P
(43) Date of publication of application: 14.05.2014
(73) Proprietor: Centre Léon Bérard, 69008 Lyon (FR); Centre National de la Recherche Scientifique (C.N.R.S.), 75016 Paris (FR); Université Claude Bernard Lyon 1, 69100 Villeurbanne (FR); INSTITUT NATIONAL DE LA SANTE ET DE LA RECHERCHE MEDICALE (INSERM), 75013 Paris (FR)
(72) Inventor: BERNARD, David, F-38460 Trept (FR); AUGERT, Arnaud, 98102 Seattle, WA (US); LAMBEAU, Gérard, F-06530 Cabris (FR); GIRARD, Chistophe, F-06100 Nice (FR); VINDRIEUX, David, F-42100 Saint-etienne (FR)
(74) Representative: Regimbeau
(86) International application number: PCT/EP2012/063272
(87) International publication number: WO 2013/007640

(56) References cited:
- WO-A2-2005/104810
- US-A1- 2011 097 732

### SEQUENCE LISTING

<110> Centre Léon Bérard CENTRE NATIONAL DE LA RECHERCHE SCIENTIFIQUE (CNRS) UNIVERSITE CLAUDE BERNARD LYON 1
<120> PLA2R1 as anti-tumoral compound and as biomarker for the detection of cancer
<130> D29662
<150> EP 11305885.3
   <151> 2011-07-08
<150> US 61/507,887
   <151> 2011-07-08
<160> 16
<170> PatentIn version 3.3
<210> 1
   <211> 4392
   <212> DNA
   <213> Homo sapiens
<220>
   <221> CDS
   <222> (1)..(4389)
<400> 1
   atg ctg ctg tcg ccg tcg ctg ctg ctg ctg ctg ctg ctg ggg gcg ccg        48
   cgg ggc tgc gcc gag ggt gtg gcg gcg gcg ctt acc ccc gag cgg ctc        96
   ctg gag tgg cag gat aaa gga ata ttt gtt atc caa agt gag agt ctc       144
   aag aaa tgc att caa gca ggt aaa tcg gtt ctg acc ctg gag aac tgc       192
   aag caa gca aac aag cac atg ctg tgg aaa tgg gtt tca aac cat ggc       240
   ctc ttt aac ata gga ggc agc ggt tgc ctg ggc ctg aat ttc tcc gcc       288
   cca gag cag cca tta agc tta tat gaa tgt gac tcc acc ctc gtt tcc       336
   tta cgg tgg cgc tgt aac agg aag atg atc aca ggc ccg ctg cag tac       384
   tct gtc cag gtg gcg cat gac aac aca gtg gtg gcc tca cgg aag tat        432
   att cat aag tgg att tct tat ggg tca ggt ggt gga gac att tgt gaa        480
   tat cta cac aaa gat ttg cat aca atc aaa ggg aac acc cac ggg atg        528
   ccg tgt atg ttt ccc ttc cag tat aac cat cag tgg cat cat gaa tgt        576
   acc cgt gaa ggt cgg gaa gat gac tta ctg tgg tgt gcc acg aca agc        624
   cgt tat gaa aga gat gaa aag tgg gga ttt tgc cct gat ccc acc tct        672
   gca gaa gta ggt tgt gat act att tgg gag aag gac ctc aat tca cac        720
   att tgc tac cag ttc aac ctg ctt tca tct ctc tct tgg agt gag gca        768
   cat tct tca tgc cag atg caa gga ggt acg ctg tta agt att aca gat        816
   gaa act gaa gaa aat ttc ata agg gag cac atg agc agt aaa aca gtg        864
   gag gtg tgg gtg ggc ctc aat cag ctg gat gaa gac gct ggc tgg cag        912
   tgg tct gat gga acg ccg ctc aac tat ctg aat tgg agc cca gag gta        960
   aat ttt gag cca ttt gtt gaa gat cac tgt gga aca ttt agt tca ttt       1008
   atg cca agt gcc tgg agg agt cgg gat tgt gag tcc acc ttg cca tat       1056
   ata tgt aaa aaa tat cta aac cac att gat cat gaa ata gtt gaa aaa       1104
   gat gcg tgg aaa tat tat gct acc cac tgt gag cct ggc tgg aat ccc       1152
   tac aat cgt aat tgc tac aaa ctt cag aaa gaa gaa aag acc tgg cat      1200
   gag gct ctg cgt tct tgt cag gct gat aac agt gca tta ata gac ata      1248
   acc tca tta gca gag gtg gag ttt ctt gta acc ctc ctt gga gat gaa      1296
   aat gca tca gaa aca tgg att ggt ttg agc agc aat aaa att cca gtt      1344
   tcc ttt gaa tgg tct aat gac tct tca gtc atc ttt act aat tgg cac      1392
   aca ctt gag ccc cac att ttt cca aat aga agc cag ctg tgt gtc tca      1440
   gca gag cag tct gag gga cac tgg aaa gtc aaa aat tgt gaa gaa aga      1488
   ctt ttt tac att tgt aaa aaa gca ggc cat gtc ctc tct gat gct gaa      1536
   tca gga tgt caa gag gga tgg gag aga cat ggt gga ttc tgt tac aaa      1584
   att gac aca gtc ctt cga agc ttt gac caa gct tcc agc ggt tat tac      1632
   tgt cct cct gca ctt gta acc att aca aac agg ttt gaa cag gct ttt      1680
   att acc agt ttg atc agt agt gtg gta aaa atg aag gac agt tat ttt      1728
   tgg ata gct ctt cag gac caa aat gat acg gga gaa tac act tgg aag      1776
   cca gta ggg cag aaa ccc gag ccg gtg cag tac aca cac tgg aac aca      1824
   cac cag ccg cgc tac agt ggt ggc tgt gtt gcc atg cga gga agg cat      1872
   cca ctt ggt cgc tgg gaa gtg aag cac tgt cgg cac ttt aag gca atg      1920
   tcc ttg tgc aag cag cca gtt gaa aat cag gaa aaa gca gag tat gaa      1968
   gag aga tgg ccc ttt cac ccc tgc tat ttg gac tgg gag tca gag cct      2016
   ggt ctg gcc agt tgc ttc aag gta ttt cat agt gaa aaa gtt ctg atg      2064
   aaa aga aca tgg aga gaa gct gaa gca ttt tgc gaa gaa ttt gga gct      2112
   cat ctt gca agc ttt gcc cat att gag gaa gag aat ttt gtg aat gag      2160
   ctc tta cat tca aaa ttt aat tgg aca gaa gaa agg cag ttc tgg att      2208
   gga ttt aat aaa aga aac cca ctg aat gcc ggc tca tgg gag tgg tct      2256
   gat aga act cct gtt gtc tct tcg ttt tta gac aac act tat ttt gga      2304
   gaa gat gca aga aac tgt gct gtt tat aag gca aac aaa aca ttg ctg      2352
   ccc tta cac tgt ggt tcc aaa cgt gaa tgg ata tgc aaa atc cca aga      2400
   gat gtg aaa ccc aag att ccg ttc tgg tac cag tac gat gta ccc tgg      2448
   ctc ttt tat cag gat gca gaa tac ctt ttt cat acc ttt gcc tca gaa      2496
   tgg ttg aac ttt gag ttt gtc tgt agc tgg ctg cac agt gat ctt ctc      2544
   aca att cat tct gca cat gag caa gaa ttc atc cac agc aaa ata aaa      2592
   gcg cta tca aag tat ggt gca agt tgg tgg att gga ctt caa gaa gaa      2640
   aga gcc aat gat gaa ttt cgc tgg aga gat gga aca cca gtg ata tac      2688
   cag aac tgg gac aca gga aga gaa aga act gtg aat aat cag agc cag      2736
   aga tgt ggc ttt att tct tct ata aca gga ctc tgg ggt agt gaa gag      2784
   tgt tca gtt tct atg cct agt atc tgt aag cga aaa aag gtt tgg ctc      2832
   ata gag aaa aag aaa gat aca cca aaa caa cat gga acg tgt ccc aaa      2880
   gga tgg cta tat ttt aac tat aag tgc ctt ctg ctg aat atc ccc aaa      2928
   gac cca agc agt tgg aag aac tgg acg cat gct caa cat ttc tgt gct      2976
   gaa gaa ggg ggg acc ctg gtc gcc  att gaa agt gag gtg  gag caa gct    3024
   ttc att  act atg aat ctt ttt  ggc cag acc acc agt  gtg tgg ata       3069
   ggt tta  caa aat gat gat tat  gaa aca tgg cta aat  gga aag cct       3114
   gtg gta  tat tct aac tgg tct  cca ttt gat ata ata  aat att cca       3159
   agt cac  aat acc act gaa gtt  cag aaa cac att cct  ctc tgt gcc       3204
   tta ctc  tca agt aat cct aat  ttt cat ttc act gga  aaa tgg tat       3249
   ttt gaa  gac tgt gga aag gaa  ggc tat ggg ttt gtt  tgt gaa aaa       3294
   atg caa  gat act tct gga cac  ggt gta aat aca tct  gat atg tat       3339
   cca atg  ccc aat acc tta gaa  tat gga aac aga act  tac aaa ata       3384
   att aat  gca aat atg act tgg  tat gca gca ata aaa  acc tgc ctg       3429
   atg cac  aaa gca caa ctg gtc  agc atc aca gac cag  tat cac cag       3474
   tcc ttc  ctc act gtt gtc ctc  aac cgg cta gga tat  gcc cac tgg       3519
   att gga  ctg ttc acc aca gat  aat ggt ctt aat ttt  gac tgg tct       3564
   gat ggc  acc aaa tct tct ttc  act ttt tgg aaa gat  gag gag tcc       3609
   tcc ctc  ctt ggt gac tgc gtt  ttt gcc gac agc aac  gga cgc tgg       3654
   cat agc  aca gcc tgc gag tca  ttt ctg caa ggt gcc  att tgt cat       3699
   gtg cca  cct gaa aca aga caa  tct gaa cac cca gag  ttg tgc tca       3744
   gaa aca  tct att ccc tgg ata  aaa ttt aaa agt aat  tgc tac agt       3789
   ttt tct  aca gtc cta gac agt  atg agt ttt gag gct  gct cat gaa       3834
   ttt tgc  aaa aag gaa ggt tct  aat ctt tta aca atc  aag gat gag       3879
   gct gaa  aat gca ttt ctc cta  gaa gag ctg ttt gct  ttt ggt tct       3924
   tct gtc  cag atg gtt tgg ttg  aat gct caa ttt gat  ggt aac aat       3969
   gaa acc  ata aag tgg ttt gat  gga act ccc aca gac  cag tca aac       4014
   tgg ggc  att cgg aag cca gac  aca gac tac ttc aag  ccc cat cat       4059
   tgt gtt  gcc ttg agg atc cct  gaa gga tta tgg cag  cta tcc ccg       4104
   tgt caa  gaa aaa aaa ggc ttt  ata tgt aaa atg gag  gca gat att      4149
   cac act  gca gag gcg ctg cca  gaa aaa gga cca agt  cac agc atc      4194
   att cct  ctt gcg gtt gta ctg  aca ctg ata gtc att  gtg gcc att      4239
   tgc aca  ctt tcc ttc tgc ata  tac aag cat aac ggt  ggc ttc ttc      4284
   agg aga  ctt gca ggg ttt cgg  aat cct tac tat cct  gca acc aac      4329
   ttt agt  aca gta tat tta gaa  gaa aat att ctc att  tct gat ctt      4374
   gag aag  agt gac caa taa                                           4392
<210> 2
   <211> 1463
   <212> PRT
   <213> Homo sapiens
<400> 2
<210> 3
   <211> 3969
   <212> DNA
   <213> Homo sapiens
<220>
   <221> CDS
   <222> (1)..(3966)
<400> 3
   atg ctg ctg tcg ccg tcg ctg ctg ctg ctg ctg ctg ctg ggg gcg ccg        48
   Met Leu Leu Ser Pro Ser Leu Leu Leu Leu Leu Leu Leu Gly Ala Pro
   1               5                   10                  15
   cgg ggc tgc gcc gag ggt gtg gcg gcg gcg ctt acc ccc gag cgg ctc        96
   Arg Gly Cys Ala Glu Gly Val Ala Ala Ala Leu Thr Pro Glu Arg Leu
               20                  25                  30
   ctg gag tgg cag gat aaa gga ata ttt gtt atc caa agt gag agt ctc       144
   Leu Glu Trp Gln Asp Lys Gly Ile Phe Val Ile Gln Ser Glu Ser Leu
           35                  40                  45
   aag aaa tgc att caa gca ggt aaa tcg gtt ctg acc ctg gag aac tgc       192
   Lys Lys Cys Ile Gln Ala Gly Lys Ser Val Leu Thr Leu Glu Asn Cys
       50                  55                  60
   aag caa gca aac aag cac atg ctg tgg aaa tgg gtt tca aac cat ggc      240
   Lys Gln Ala Asn Lys His Met Leu Trp Lys Trp Val Ser Asn His Gly
   65                  70                  75                  80
   ctc ttt aac ata gga ggc agc ggt tgc ctg ggc ctg aat ttc tcc gcc      288
   Leu Phe Asn Ile Gly Gly Ser Gly Cys Leu Gly Leu Asn Phe Ser Ala
                   85                  90                  95
   cca gag cag cca tta agc tta tat gaa tgt gac tcc acc ctc gtt tcc      336
   Pro Glu Gln Pro Leu Ser Leu Tyr Glu Cys Asp Ser Thr Leu Val Ser
               100                 105                 110
   tta cgg tgg cgc tgt aac agg aag atg atc aca ggc ccg ctg cag tac      384
   Leu Arg Trp Arg Cys Asn Arg Lys Met Ile Thr Gly Pro Leu Gln Tyr
           115                 120                 125
   tct gtc cag gtg gcg cat gac aac aca gtg gtg gcc tca cgg aag tat      432
   Ser Val Gln Val Ala His Asp Asn Thr Val Val Ala Ser Arg Lys Tyr
       130                 135                 140
   att cat aag tgg att tct tat ggg tca ggt ggt gga gac att tgt gaa      480
   Ile His Lys Trp Ile Ser Tyr Gly Ser Gly Gly Gly Asp Ile Cys Glu
   145                 150                 155                 160
   tat cta cac aaa gat ttg cat aca atc aaa ggg aac acc cac ggg atg      528
   Tyr Leu His Lys Asp Leu His Thr Ile Lys Gly Asn Thr His Gly Met
                   165                 170                 175
   ccg tgt atg ttt ccc ttc cag tat aac cat cag tgg cat cat gaa tgt      576
   Pro Cys Met Phe Pro Phe Gln Tyr Asn His Gln Trp His His Glu Cys
               180                 185                 190
   acc cgt gaa ggt cgg gaa gat gac tta ctg tgg tgt gcc acg aca agc      624
   Thr Arg Glu Gly Arg Glu Asp Asp Leu Leu Trp Cys Ala Thr Thr Ser
           195                 200                 205
   cgt tat gaa aga gat gaa aag tgg gga ttt tgc cct gat ccc acc tct      672
   Arg Tyr Glu Arg Asp Glu Lys Trp Gly Phe Cys Pro Asp Pro Thr Ser
       210                 215                 220
   gca gaa gta ggt tgt gat act att tgg gag aag gac ctc aat tca cac      720
   Ala Glu Val Gly Cys Asp Thr Ile Trp Glu Lys Asp Leu Asn Ser His
   225                 230                 235                 240
   att tgc tac cag ttc aac ctg ctt tca tct ctc tct tgg agt gag gca      768
   Ile Cys Tyr Gln Phe Asn Leu Leu Ser Ser Leu Ser Trp Ser Glu Ala
                   245                 250                 255
   cat tct tca tgc cag atg caa gga ggt acg ctg tta agt att aca gat      816
   His Ser Ser Cys Gln Met Gln Gly Gly Thr Leu Leu Ser Ile Thr Asp
               260                 265                 270
   gaa act gaa gaa aat ttc ata agg gag cac atg agc agt aaa aca gtg      864
   Glu Thr Glu Glu Asn Phe Ile Arg Glu His Met Ser Ser Lys Thr Val
           275                 280                 285
   gag gtg tgg gtg ggc ctc aat cag ctg gat gaa gac gct ggc tgg cag      912
   Glu Val Trp Val Gly Leu Asn Gln Leu Asp Glu Asp Ala Gly Trp Gln
       290                 295                 300
   tgg tct gat gga acg ccg ctc aac tat ctg aat tgg agc cca gag gta      960
   Trp Ser Asp Gly Thr Pro Leu Asn Tyr Leu Asn Trp Ser Pro Glu Val
   305                 310                 315                 320
   aat ttt gag cca ttt gtt gaa gat cac tgt gga aca ttt agt tca ttt      1008
   Asn Phe Glu Pro Phe Val Glu Asp His Cys Gly Thr Phe Ser Ser Phe
                   325                 330                 335
   atg cca agt gcc tgg agg agt cgg gat tgt gag tcc acc ttg cca tat      1056
   Met Pro Ser Ala Trp Arg Ser Arg Asp Cys Glu Ser Thr Leu Pro Tyr
               340                 345                 350
   ata tgt aaa aaa tat cta aac cac att gat cat gaa ata gtt gaa aaa      1104
   Ile Cys Lys Lys Tyr Leu Asn His Ile Asp His Glu Ile Val Glu Lys
           355                 360                 365
   gat gcg tgg aaa tat tat gct acc cac tgt gag cct ggc tgg aat ccc      1152
   Asp Ala Trp Lys Tyr Tyr Ala Thr His Cys Glu Pro Gly Trp Asn Pro
       370                 375                 380
   tac aat cgt aat tgc tac aaa ctt cag aaa gaa gaa aag acc tgg cat      1200
   Tyr Asn Arg Asn Cys Tyr Lys Leu Gln Lys Glu Glu Lys Thr Trp His
   385                 390                 395                 400
   gag gct ctg cgt tct tgt cag gct gat aac agt gca tta ata gac ata      1248
   Glu Ala Leu Arg Ser Cys Gln Ala Asp Asn Ser Ala Leu Ile Asp Ile
                   405                 410                 415
   acc tca tta gca gag gtg gag ttt ctt gta acc ctc ctt gga gat gaa      1296
   Thr Ser Leu Ala Glu Val Glu Phe Leu Val Thr Leu Leu Gly Asp Glu
               420                 425                 430
   aat gca tca gaa aca tgg att ggt ttg agc agc aat aaa att cca gtt      1344
   Asn Ala Ser Glu Thr Trp Ile Gly Leu Ser Ser Asn Lys Ile Pro Val
           435                 440                 445
   tcc ttt gaa tgg tct aat gac tct tca gtc atc ttt act aat tgg cac      1392
   Ser Phe Glu Trp Ser Asn Asp Ser Ser Val Ile Phe Thr Asn Trp His
       450                 455                 460
   aca ctt gag ccc cac att ttt cca aat aga agc cag ctg tgt gtc tca      1440
   Thr Leu Glu Pro His Ile Phe Pro Asn Arg Ser Gln Leu Cys Val Ser
   465                 470                 475                 480
   gca gag cag tct gag gga cac tgg aaa gtc aaa aat tgt gaa gaa aga      1488
   Ala Glu Gln Ser Glu Gly His Trp Lys Val Lys Asn Cys Glu Glu Arg
                   485                 490                 495
   ctt ttt tac att tgt aaa aaa gca ggc cat gtc ctc tct gat gct gaa      1536
   Leu Phe Tyr Ile Cys Lys Lys Ala Gly His Val Leu Ser Asp Ala Glu
               500                 505                 510
   tca gga tgt caa gag gga tgg gag aga cat ggt gga ttc tgt tac aaa      1584
   Ser Gly Cys Gln Glu Gly Trp Glu Arg His Gly Gly Phe Cys Tyr Lys
           515                 520                 525
   att gac aca gtc ctt cga agc ttt gac caa gct tcc agc ggt tat tac      1632
   Ile Asp Thr Val Leu Arg Ser Phe Asp Gln Ala Ser Ser Gly Tyr Tyr
       530                 535                 540
   tgt cct cct gca ctt gta acc att aca aac agg ttt gaa cag gct ttt      1680
   Cys Pro Pro Ala Leu Val Thr Ile Thr Asn Arg Phe Glu Gln Ala Phe
   545                 550                 555                 560
   att acc agt ttg atc agt agt gtg gta aaa atg aag gac agt tat ttt      1728
   Ile Thr Ser Leu Ile Ser Ser Val Val Lys Met Lys Asp Ser Tyr Phe
   565 570 575
   tgg ata gct ctt cag gac caa aat gat acg gga gaa tac act tgg aag 1776
   Trp Ile Ala Leu Gln Asp Gln Asn Asp Thr Gly Glu Tyr Thr Trp Lys
   580 585 590
   cca gta ggg cag aaa ccc gag ccg gtg cag tac aca cac tgg aac aca 1824
   Pro Val Gly Gln Lys Pro Glu Pro Val Gln Tyr Thr His Trp Asn Thr
   595 600 605
   cac cag ccg cgc tac agt ggt ggc tgt gtt gcc atg cga gga agg cat 1872
   His Gln Pro Arg Tyr Ser Gly Gly Cys Val Ala Met Arg Gly Arg His
   610 615 620
   cca ctt ggt cgc tgg gaa gtg aag cac tgt cgg cac ttt aag gca atg 1920
   Pro Leu Gly Arg Trp Glu Val Lys His Cys Arg His Phe Lys Ala Met
   625 630 635 640
   tcc ttg tgc aag cag cca gtt gaa aat cag gaa aaa gca gag tat gaa 1968
   Ser Leu Cys Lys Gln Pro Val Glu Asn Gln Glu Lys Ala Glu Tyr Glu
   645 650 655
   gag aga tgg ccc ttt cac ccc tgc tat ttg gac tgg gag tca gag cct 2016
   Glu Arg Trp Pro Phe His Pro Cys Tyr Leu Asp Trp Glu Ser Glu Pro
   660 665 670
   ggt ctg gcc agt tgc ttc aag gta ttt cat agt gaa aaa gtt ctg atg 2064
   Gly Leu Ala Ser Cys Phe Lys Val Phe His Ser Glu Lys Val Leu Met
   675 680 685
   aaa aga aca tgg aga gaa gct gaa gca ttt tgc gaa gaa ttt gga gct 2112
   Lys Arg Thr Trp Arg Glu Ala Glu Ala Phe Cys Glu Glu Phe Gly Ala
   690 695 700
   cat ctt gca agc ttt gcc cat att gag gaa gag aat ttt gtg aat gag 2160
   His Leu Ala Ser Phe Ala His Ile Glu Glu Glu Asn Phe Val Asn Glu
   705 710 715 720
   ctc tta cat tca aaa ttt aat tgg aca gaa gaa agg cag ttc tgg att 2208
   Leu Leu His Ser Lys Phe Asn Trp Thr Glu Glu Arg Gln Phe Trp Ile
   725 730 735
   gga ttt aat aaa aga aac cca ctg aat gcc ggc tca tgg gag tgg tct 2256
   Gly Phe Asn Lys Arg Asn Pro Leu Asn Ala Gly Ser Trp Glu Trp Ser
   740 745 750
   gat aga act cct gtt gtc tct tcg ttt tta gac aac act tat ttt gga 2304
   Asp Arg Thr Pro Val Val Ser Ser Phe Leu Asp Asn Thr Tyr Phe Gly
   755 760 765
   gaa gat gca aga aac tgt gct gtt tat aag gca aac aaa aca ttg ctg 2352
   Glu Asp Ala Arg Asn Cys Ala Val Tyr Lys Ala Asn Lys Thr Leu Leu
   770 775 780
   ccc tta cac tgt ggt tcc aaa cgt gaa tgg ata tgc aaa atc cca aga 2400
   Pro Leu His Cys Gly Ser Lys Arg Glu Trp Ile Cys Lys Ile Pro Arg
   785 790 795 800
   gat gtg aaa ccc aag att ccg ttc tgg tac cag tac gat gta ccc tgg 2448
   Asp Val Lys Pro Lys Ile Pro Phe Trp Tyr Gln Tyr Asp Val Pro Trp
   805 810 815
   ctc ttt tat cag gat gca gaa tac ctt ttt cat acc ttt gcc tca gaa 2496
   Leu Phe Tyr Gln Asp Ala Glu Tyr Leu Phe His Thr Phe Ala Ser Glu
               820                 825                 830
   tgg ttg aac ttt gag ttt gtc tgt agc tgg ctg cac agt gat ctt ctc     2544
   Trp Leu Asn Phe Glu Phe Val Cys Ser Trp Leu His Ser Asp Leu Leu
           835                 840                 845
   aca att cat tct gca cat gag caa gaa ttc atc cac agc aaa ata aaa     2592
   Thr Ile His Ser Ala His Glu Gln Glu Phe Ile His Ser Lys Ile Lys
       850                 855                 860
   gcg cta tca aag tat ggt gca agt tgg tgg att gga ctt caa gaa gaa     2640
   Ala Leu Ser Lys Tyr Gly Ala Ser Trp Trp Ile Gly Leu Gln Glu Glu
   865                 870                 875                 880
   aga gcc aat gat gaa ttt cgc tgg aga gat gga aca cca gtg ata tac     2688
   Arg Ala Asn Asp Glu Phe Arg Trp Arg Asp Gly Thr Pro Val Ile Tyr
                   885                 890                 895
   cag aac tgg gac aca gga aga gaa aga act gtg aat aat cag agc cag     2736
   Gln Asn Trp Asp Thr Gly Arg Glu Arg Thr Val Asn Asn Gln Ser Gln
               900                 905                 910
   aga tgt ggc ttt att tct tct ata aca gga ctc tgg ggt agt gaa gag     2784
   Arg Cys Gly Phe Ile Ser Ser Ile Thr Gly Leu Trp Gly Ser Glu Glu
           915                 920                 925
   tgt tca gtt tct atg cct agt atc tgt aag cga aaa aag gtt tgg ctc     2832
   Cys Ser Val Ser Met Pro Ser Ile Cys Lys Arg Lys Lys Val Trp Leu
       930                 935                 940
   ata gag aaa aag aaa gat aca cca aaa caa cat gga acg tgt ccc aaa     2880
   Ile Glu Lys Lys Lys Asp Thr Pro Lys Gln His Gly Thr Cys Pro Lys
   945                 950                 955                 960
   gga tgg cta tat ttt aac tat aag tgc ctt ctg ctg aat atc ccc aaa     2928
   Gly Trp Leu Tyr Phe Asn Tyr Lys Cys Leu Leu Leu Asn Ile Pro Lys
                   965                 970                 975
   gac cca agc agt tgg aag aac tgg acg cat gct caa cat ttc tgt gct     2976
   Asp Pro Ser Ser Trp Lys Asn Trp Thr His Ala Gln His Phe Cys Ala
               980                 985                 990
   gaa gaa ggg ggg acc ctg gtc gcc  att gaa agt gag gtg  gag caa gct   3024
   Glu Glu Gly Gly Thr Leu Val Ala  Ile Glu Ser Glu Val  Glu Gln Ala
           995                 1000                1005
   ttc att  act atg aat ctt ttt  ggc cag acc acc agt  gtg tgg ata     3069
   Phe Ile  Thr Met Asn Leu Phe  Gly Gln Thr Thr Ser  Val Trp Ile
       1010                 1015                 1020
   ggt tta  caa aat gat gat tat  gaa aca tgg cta aat  gga aag cct     3114
   Gly Leu  Gln Asn Asp Asp Tyr  Glu Thr Trp Leu Asn  Gly Lys Pro
       1025                 1030                 1035
   gtg gta  tat tct aac tgg tct  cca ttt gat ata ata  aat att cca     3159
   Val Val  Tyr Ser Asn Trp Ser  Pro Phe Asp Ile Ile  Asn Ile Pro
       1040                 1045                 1050
   agt cac  aat acc act gaa gtt  cag aaa cac att cct  ctc tgt gcc     3204
   Ser His  Asn Thr Thr Glu Val  Gln Lys His Ile Pro  Leu Cys Ala
       1055                 1060                 1065
   tta ctc  tca agt aat cct aat  ttt cat ttc act gga  aaa tgg tat       3249
   Leu Leu  Ser Ser Asn Pro Asn  Phe His Phe Thr Gly  Lys Trp Tyr
       1070                 1075                 1080
   ttt gaa  gac tgt gga aag gaa  ggc tat ggg ttt gtt  tgt gaa aaa       3294
   Phe Glu  Asp Cys Gly Lys Glu  Gly Tyr Gly Phe Val  Cys Glu Lys
       1085                 1090                 1095
   atg caa  gat act tct gga cac  ggt gta aat aca tct  gat atg tat       3339
   Met Gln  Asp Thr Ser Gly His  Gly Val Asn Thr Ser  Asp Met Tyr
       1100                 1105                 1110
   cca atg  ccc aat acc tta gaa  tat gga aac aga act  tac aaa ata       3384
   Pro Met  Pro Asn Thr Leu Glu  Tyr Gly Asn Arg Thr  Tyr Lys Ile
       1115                 1120                 1125
   att aat  gca aat atg act tgg  tat gca gca ata aaa  acc tgc ctg       3429
   Ile Asn  Ala Asn Met Thr Trp  Tyr Ala Ala Ile Lys  Thr Cys Leu
       1130                 1135                 1140
   atg cac  aaa gca caa ctg gtc  agc atc aca gac cag  tat cac cag       3474
   Met His  Lys Ala Gln Leu Val  Ser Ile Thr Asp Gln  Tyr His Gln
       1145                 1150                 1155
   tcc ttc  ctc act gtt gtc ctc  aac cgg cta gga tat  gcc cac tgg       3519
   Ser Phe  Leu Thr Val Val Leu  Asn Arg Leu Gly Tyr  Ala His Trp
       1160                 1165                 1170
   att gga  ctg ttc acc aca gat  aat ggt ctt aat ttt  gac tgg tct       3564
   Ile Gly  Leu Phe Thr Thr Asp  Asn Gly Leu Asn Phe  Asp Trp Ser
       1175                 1180                 1185
   gat ggc  acc aaa tct tct ttc  act ttt tgg aaa gat  gag gag tcc       3609
   Asp Gly  Thr Lys Ser Ser Phe  Thr Phe Trp Lys Asp  Glu Glu Ser
       1190                 1195                 1200
   tcc ctc  ctt ggt gac tgc gtt  ttt gcc gac agc aac  gga cgc tgg       3654
   Ser Leu  Leu Gly Asp Cys Val  Phe Ala Asp Ser Asn  Gly Arg Trp
       1205                 1210                 1215
   cat agc  aca gcc tgc gag tca  ttt ctg caa ggt gcc  att tgt cat       3699
   His Ser  Thr Ala Cys Glu Ser  Phe Leu Gln Gly Ala  Ile Cys His
       1220                 1225                 1230
   gtg cca  cct gaa aca aga caa  tct gaa cac cca gag  ttg tgc tca       3744
   Val Pro  Pro Glu Thr Arg Gln  Ser Glu His Pro Glu  Leu Cys Ser
       1235                 1240                 1245
   gaa aca  tct att ccc tgg ata  aaa ttt aaa agt aat  tgc tac agt       3789
   Glu Thr  Ser Ile Pro Trp Ile  Lys Phe Lys Ser Asn  Cys Tyr Ser
       1250                 1255                 1260
   ttt tct  aca gtc cta gac agt  atg agt ttt gag gct  gct cat gaa       3834
   Phe Ser  Thr Val Leu Asp Ser  Met Ser Phe Glu Ala  Ala His Glu
       1265                 1270                 1275
   ttt tgc  aaa aag gaa ggt tct  aat ctt tta aca atc  aag gat gag       3879
   Phe Cys  Lys Lys Glu Gly Ser  Asn Leu Leu Thr Ile  Lys Asp Glu
       1280                 1285                 1290
   gct gaa  aat gca ttt ctc cta  gaa gag ctg ttt gct  ttt ggt tct       3924
   Ala Glu  Asn Ala Phe Leu Leu  Glu Glu Leu Phe Ala  Phe Gly Ser
       1295                 1300                 1305
   tct gtc  cag atg gtt tgg ttg  aat gct caa ttt gat  ggt aac taa      3969
   Ser Val  Gln Met Val Trp Leu  Asn Ala Gln Phe Asp  Gly Asn
       1310                 1315                 1320
<210> 4
   <211> 1322
   <212> PRT
   <213> Homo sapiens
<400> 4
   Met Leu Leu Ser Pro Ser Leu Leu Leu Leu Leu Leu Leu Gly Ala Pro
   1               5                   10                  15
   Arg Gly Cys Ala Glu Gly Val Ala Ala Ala Leu Thr Pro Glu Arg Leu
               20                  25                  30
   Leu Glu Trp Gln Asp Lys Gly Ile Phe Val Ile Gln Ser Glu Ser Leu
           35                  40                  45
   Lys Lys Cys Ile Gln Ala Gly Lys Ser Val Leu Thr Leu Glu Asn Cys
       50                  55                  60
   Lys Gln Ala Asn Lys His Met Leu Trp Lys Trp Val Ser Asn His Gly
   65                  70                  75                  80
   Leu Phe Asn Ile Gly Gly Ser Gly Cys Leu Gly Leu Asn Phe Ser Ala
                   85                  90                  95
   Pro Glu Gln Pro Leu Ser Leu Tyr Glu Cys Asp Ser Thr Leu Val Ser
               100                 105                 110
   Leu Arg Trp Arg Cys Asn Arg Lys Met Ile Thr Gly Pro Leu Gln Tyr
           115                 120                 125
   Ser Val Gln Val Ala His Asp Asn Thr Val Val Ala Ser Arg Lys Tyr
       130                 135                 140
   Ile His Lys Trp Ile Ser Tyr Gly Ser Gly Gly Gly Asp Ile Cys Glu
   145                 150                 155                 160
   Tyr Leu His Lys Asp Leu His Thr Ile Lys Gly Asn Thr His Gly Met
                   165                 170                 175
   Pro Cys Met Phe Pro Phe Gln Tyr Asn His Gln Trp His His Glu Cys
               180                 185                 190
   Thr Arg Glu Gly Arg Glu Asp Asp Leu Leu Trp Cys Ala Thr Thr Ser
           195                 200                 205
   Arg Tyr Glu Arg Asp Glu Lys Trp Gly Phe Cys Pro Asp Pro Thr Ser
       210                 215                 220
   Ala Glu Val Gly Cys Asp Thr Ile Trp Glu Lys Asp Leu Asn Ser His
   225                 230                 235                 240
   Ile Cys Tyr Gln Phe Asn Leu Leu Ser Ser Leu Ser Trp Ser Glu Ala
                   245                 250                 255
   His Ser Ser Cys Gln Met Gln Gly Gly Thr Leu Leu Ser Ile Thr Asp
               260                 265                 270
   Glu Thr Glu Glu Asn Phe Ile Arg Glu His Met Ser Ser Lys Thr Val
           275                 280                 285
   Glu Val Trp Val Gly Leu Asn Gln Leu Asp Glu Asp Ala Gly Trp Gln
       290                 295                 300
   Trp Ser Asp Gly Thr Pro Leu Asn Tyr Leu Asn Trp Ser Pro Glu Val
   305                 310                 315                 320
   Asn Phe Glu Pro Phe Val Glu Asp His Cys Gly Thr Phe Ser Ser Phe
                   325                 330                 335
   Met Pro Ser Ala Trp Arg Ser Arg Asp Cys Glu Ser Thr Leu Pro Tyr
               340                 345                 350
   Ile Cys Lys Lys Tyr Leu Asn His Ile Asp His Glu Ile Val Glu Lys
           355                 360                 365
   Asp Ala Trp Lys Tyr Tyr Ala Thr His Cys Glu Pro Gly Trp Asn Pro
       370                 375                 380
   Tyr Asn Arg Asn Cys Tyr Lys Leu Gln Lys Glu Glu Lys Thr Trp His
   385                 390                 395                 400
   Glu Ala Leu Arg Ser Cys Gln Ala Asp Asn Ser Ala Leu Ile Asp Ile
                   405                 410                 415
   Thr Ser Leu Ala Glu Val Glu Phe Leu Val Thr Leu Leu Gly Asp Glu
               420                 425                 430
   Asn Ala Ser Glu Thr Trp Ile Gly Leu Ser Ser Asn Lys Ile Pro Val
           435                 440                 445
   Ser Phe Glu Trp Ser Asn Asp Ser Ser Val Ile Phe Thr Asn Trp His
       450                 455                 460
   Thr Leu Glu Pro His Ile Phe Pro Asn Arg Ser Gln Leu Cys Val Ser
   465                 470                 475                 480
   Ala Glu Gln Ser Glu Gly His Trp Lys Val Lys Asn Cys Glu Glu Arg
                   485                 490                 495
   Leu Phe Tyr Ile Cys Lys Lys Ala Gly His Val Leu Ser Asp Ala Glu
               500                 505                 510
   Ser Gly Cys Gln Glu Gly Trp Glu Arg His Gly Gly Phe Cys Tyr Lys
           515                 520                 525
   Ile Asp Thr Val Leu Arg Ser Phe Asp Gln Ala Ser Ser Gly Tyr Tyr
       530                 535                 540
   Cys Pro Pro Ala Leu Val Thr Ile Thr Asn Arg Phe Glu Gln Ala Phe
   545                 550                 555                 560
   Ile Thr Ser Leu Ile Ser Ser Val Val Lys Met Lys Asp Ser Tyr Phe
                   565                 570                 575
   Trp Ile Ala Leu Gln Asp Gln Asn Asp Thr Gly Glu Tyr Thr Trp Lys
               580                 585                 590
   Pro Val Gly Gln Lys Pro Glu Pro Val Gln Tyr Thr His Trp Asn Thr
           595                 600                 605
   His Gln Pro Arg Tyr Ser Gly Gly Cys Val Ala Met Arg Gly Arg His
       610                 615                 620
   Pro Leu Gly Arg Trp Glu Val Lys His Cys Arg His Phe Lys Ala Met
   625                 630                 635                 640
   Ser Leu Cys Lys Gln Pro Val Glu Asn Gln Glu Lys Ala Glu Tyr Glu
                   645                 650                 655
   Glu Arg Trp Pro Phe His Pro Cys Tyr Leu Asp Trp Glu Ser Glu Pro
               660                 665                 670
   Gly Leu Ala Ser Cys Phe Lys Val Phe His Ser Glu Lys Val Leu Met
           675                 680                 685
   Lys Arg Thr Trp Arg Glu Ala Glu Ala Phe Cys Glu Glu Phe Gly Ala
       690                 695                 700
   His Leu Ala Ser Phe Ala His Ile Glu Glu Glu Asn Phe Val Asn Glu
   705 710 715 720
   Leu Leu His Ser Lys Phe Asn Trp Thr Glu Glu Arg Gln Phe Trp Ile
   725 730 735
   Gly Phe Asn Lys Arg Asn Pro Leu Asn Ala Gly Ser Trp Glu Trp Ser
   740 745 750
   Asp Arg Thr Pro Val Val Ser Ser Phe Leu Asp Asn Thr Tyr Phe Gly
   755 760 765
   Glu Asp Ala Arg Asn Cys Ala Val Tyr Lys Ala Asn Lys Thr Leu Leu
   770 775 780
   Pro Leu His Cys Gly Ser Lys Arg Glu Trp Ile Cys Lys Ile Pro Arg
   785 790 795 800
   Asp Val Lys Pro Lys Ile Pro Phe Trp Tyr Gln Tyr Asp Val Pro Trp
   805 810 815
   Leu Phe Tyr Gln Asp Ala Glu Tyr Leu Phe His Thr Phe Ala Ser Glu
   820 825 830
   Trp Leu Asn Phe Glu Phe Val Cys Ser Trp Leu His Ser Asp Leu Leu
   835 840 845
   Thr Ile His Ser Ala His Glu Gln Glu Phe Ile His Ser Lys Ile Lys
   850 855 860
   Ala Leu Ser Lys Tyr Gly Ala Ser Trp Trp Ile Gly Leu Gln Glu Glu
   865 870 875 880
   Arg Ala Asn Asp Glu Phe Arg Trp Arg Asp Gly Thr Pro Val Ile Tyr
   885 890 895
   Gln Asn Trp Asp Thr Gly Arg Glu Arg Thr Val Asn Asn Gln Ser Gln
   900 905 910
   Arg Cys Gly Phe Ile Ser Ser Ile Thr Gly Leu Trp Gly Ser Glu Glu
   915 920 925
   Cys Ser Val Ser Met Pro Ser Ile Cys Lys Arg Lys Lys Val Trp Leu
   930 935 940
   Ile Glu Lys Lys Lys Asp Thr Pro Lys Gln His Gly Thr Cys Pro Lys
   945 950 955 960
   Gly Trp Leu Tyr Phe Asn Tyr Lys Cys Leu Leu Leu Asn Ile Pro Lys
                   965                 970                 975
   Asp Pro Ser Ser Trp Lys Asn Trp Thr His Ala Gln His Phe Cys Ala
               980                 985                 990
   Glu Glu Gly Gly Thr Leu Val Ala  Ile Glu Ser Glu Val  Glu Gln Ala
           995                 1000                 1005
   Phe Ile  Thr Met Asn Leu Phe  Gly Gln Thr Thr Ser  Val Trp Ile
       1010                 1015                 1020
   Gly Leu  Gln Asn Asp Asp Tyr  Glu Thr Trp Leu Asn  Gly Lys Pro
       1025                 1030                 1035
   Val Val  Tyr Ser Asn Trp Ser  Pro Phe Asp Ile Ile  Asn Ile Pro
       1040                 1045                 1050
   Ser His  Asn Thr Thr Glu Val  Gln Lys His Ile Pro  Leu Cys Ala
       1055                 1060                 1065
   Leu Leu  Ser Ser Asn Pro Asn  Phe His Phe Thr Gly  Lys Trp Tyr
       1070                 1075                 1080
   Phe Glu  Asp Cys Gly Lys Glu  Gly Tyr Gly Phe Val  Cys Glu Lys
       1085                 1090                 1095
   Met Gln  Asp Thr Ser Gly His  Gly Val Asn Thr Ser  Asp Met Tyr
       1100                 1105                 1110
   Pro Met  Pro Asn Thr Leu Glu  Tyr Gly Asn Arg Thr  Tyr Lys Ile
       1115                 1120                 1125
   Ile Asn  Ala Asn Met Thr Trp  Tyr Ala Ala Ile Lys  Thr Cys Leu
       1130                 1135                 1140
   Met His  Lys Ala Gln Leu Val  Ser Ile Thr Asp Gln  Tyr His Gln
       1145                 1150                 1155
   Ser Phe  Leu Thr Val Val Leu  Asn Arg Leu Gly Tyr  Ala His Trp
       1160                 1165                 1170
   Ile Gly  Leu Phe Thr Thr Asp  Asn Gly Leu Asn Phe  Asp Trp Ser
       1175                 1180                 1185
   Asp Gly  Thr Lys Ser Ser Phe  Thr Phe Trp Lys Asp  Glu Glu Ser
       1190                 1195                 1200
   Ser Leu Leu Gly Asp Cys Val Phe Ala Asp Ser Asn Gly Arg Trp
       1205                1210                1215
   His Ser Thr Ala Cys Glu Ser Phe Leu Gln Gly Ala Ile Cys His
       1220                1225                1230
   Val Pro Pro Glu Thr Arg Gln Ser Glu His Pro Glu Leu Cys Ser
       1235                1240                1245
   Glu Thr Ser Ile Pro Trp Ile Lys Phe Lys Ser Asn Cys Tyr Ser
       1250                1255                1260
   Phe Ser Thr Val Leu Asp Ser Met Ser Phe Glu Ala Ala His Glu
       1265                1270                1275
   Phe Cys Lys Lys Glu Gly Ser Asn Leu Leu Thr Ile Lys Asp Glu
       1280                1285                1290
   Ala Glu Asn Ala Phe Leu Leu Glu Glu Leu Phe Ala Phe Gly Ser
       1295                1300                1305
   Ser Val Gln Met Val Trp Leu Asn Ala Gln Phe Asp Gly Asn
       1310                1315                1320
<210> 5
   <211> 3297
   <212> DNA
   <213> Homo sapiens
<220>
   <221> CDS
   <222> (1)..(3294)
<400> 5
   atg ctg ctg tcg ccg tcg ctg ctg ctg ctg ctg ctg ctg ggg gcg ccg        48
   Met Leu Leu Ser Pro Ser Leu Leu Leu Leu Leu Leu Leu Gly Ala Pro
   1               5                   10                  15
   cgg ggc tgc gcc gag ggt gtg gcg gcg gcg ctt acc ccc gag cgg ctc        96
   Arg Gly Cys Ala Glu Gly Val Ala Ala Ala Leu Thr Pro Glu Arg Leu
               20                  25                  30
   ctg gag tgg cag gat aaa gga ata ttt gtt atc caa agt gag agt ctc       144
   Leu Glu Trp Gln Asp Lys Gly Ile Phe Val Ile Gln Ser Glu Ser Leu
           35                  40                  45
   aag aaa tgc att caa gca ggt aaa tcg gtt ctg acc ctg gag aac tgc       192
   Lys Lys Cys Ile Gln Ala Gly Lys Ser Val Leu Thr Leu Glu Asn Cys
       50                  55                  60
   aag caa gca aac aag cac atg ctg tgg aaa tgg gtt tca aac cat ggc       240
   Lys Gln Ala Asn Lys His Met Leu Trp Lys Trp Val Ser Asn His Gly
   65                  70                  75                  80
   ctc ttt aac ata gga ggc agc ggt tgc ctg ggc ctg aat ttc tcc gcc      288
   Leu Phe Asn Ile Gly Gly Ser Gly Cys Leu Gly Leu Asn Phe Ser Ala
                   85                  90                  95
   cca gag cag cca tta agc tta tat gaa tgt gac tcc acc ctc gtt tcc      336
   Pro Glu Gln Pro Leu Ser Leu Tyr Glu Cys Asp Ser Thr Leu Val Ser
               100                 105                 110
   tta cgg tgg cgc tgt aac agg aag atg atc aca ggc ccg ctg cag tac      384
   Leu Arg Trp Arg Cys Asn Arg Lys Met Ile Thr Gly Pro Leu Gln Tyr
           115                 120                 125
   tct gtc cag gtg gcg cat gac aac aca gtg gtg gcc tca cgg aag tat      432
   Ser Val Gln Val Ala His Asp Asn Thr Val Val Ala Ser Arg Lys Tyr
       130                 135                 140
   att cat aag tgg att tct tat ggg tca ggt ggt gga gac att tgt gaa      480
   Ile His Lys Trp Ile Ser Tyr Gly Ser Gly Gly Gly Asp Ile Cys Glu
   145                 150                 155                 160
   tat cta cac aaa gat ttg cat aca atc aaa ggg aac acc cac ggg atg      528
   Tyr Leu His Lys Asp Leu His Thr Ile Lys Gly Asn Thr His Gly Met
                   165                 170                 175
   ccg tgt atg ttt ccc ttc cag tat aac cat cag tgg cat cat gaa tgt      576
   Pro Cys Met Phe Pro Phe Gln Tyr Asn His Gln Trp His His Glu Cys
               180                 185                 190
   acc cgt gaa ggt cgg gaa gat gac tta ctg tgg tgt gcc acg aca agc      624
   Thr Arg Glu Gly Arg Glu Asp Asp Leu Leu Trp Cys Ala Thr Thr Ser
           195                 200                 205
   cgt tat gaa aga gat gaa aag tgg gga ttt tgc cct gat ccc acc tct      672
   Arg Tyr Glu Arg Asp Glu Lys Trp Gly Phe Cys Pro Asp Pro Thr Ser
       210                 215                 220
   gca gaa gta ggt tgt gat act att tgg gag aag gac ctc aat tca cac      720
   Ala Glu Val Gly Cys Asp Thr Ile Trp Glu Lys Asp Leu Asn Ser His
   225                 230                 235                 240
   att tgc tac cag ttc aac ctg ctt tca tct ctc tct tgg agt gag gca      768
   Ile Cys Tyr Gln Phe Asn Leu Leu Ser Ser Leu Ser Trp Ser Glu Ala
                   245                 250                 255
   cat tct tca tgc cag atg caa gga ggt acg ctg tta agt att aca gat      816
   His Ser Ser Cys Gln Met Gln Gly Gly Thr Leu Leu Ser Ile Thr Asp
               260                 265                 270
   gaa act gaa gaa aat ttc ata agg gag cac atg agc agt aaa aca gtg      864
   Glu Thr Glu Glu Asn Phe Ile Arg Glu His Met Ser Ser Lys Thr Val
           275                 280                 285
   gag gtg tgg gtg ggc ctc aat cag ctg gat gaa gac gct ggc tgg cag      912
   Glu Val Trp Val Gly Leu Asn Gln Leu Asp Glu Asp Ala Gly Trp Gln
       290                 295                 300
   tgg tct gat gga acg ccg ctc aac tat ctg aat tgg agc cca gag gta      960
   Trp Ser Asp Gly Thr Pro Leu Asn Tyr Leu Asn Trp Ser Pro Glu Val
   305                 310                 315                 320
   aat ttt gag cca ttt gtt gaa gat cac tgt gga aca ttt agt tca ttt     1008
   Asn Phe Glu Pro Phe Val Glu Asp His Cys Gly Thr Phe Ser Ser Phe
                   325                 330                 335
   atg cca agt gcc tgg agg agt cgg gat tgt gag tcc acc ttg cca tat      1056
   Met Pro Ser Ala Trp Arg Ser Arg Asp Cys Glu Ser Thr Leu Pro Tyr
               340                 345                 350
   ata tgt aaa aaa tat cta aac cac att gat cat gaa ata gtt gaa aaa      1104
   Ile Cys Lys Lys Tyr Leu Asn His Ile Asp His Glu Ile Val Glu Lys
           355                 360                 365
   gat gcg tgg aaa tat tat gct acc cac tgt gag cct ggc tgg aat ccc      1152
   Asp Ala Trp Lys Tyr Tyr Ala Thr His Cys Glu Pro Gly Trp Asn Pro
       370                 375                 380
   tac aat cgt aat tgc tac aaa ctt cag aaa gaa gaa aag acc tgg cat      1200
   Tyr Asn Arg Asn Cys Tyr Lys Leu Gln Lys Glu Glu Lys Thr Trp His
   385                 390                 395                 400
   gag gct ctg cgt tct tgt cag gct gat aac agt gca tta ata gac ata      1248
   Glu Ala Leu Arg Ser Cys Gln Ala Asp Asn Ser Ala Leu Ile Asp Ile
                   405                 410                 415
   acc tca tta gca gag gtg gag ttt ctt gta acc ctc ctt gga gat gaa      1296
   Thr Ser Leu Ala Glu Val Glu Phe Leu Val Thr Leu Leu Gly Asp Glu
               420                 425                 430
   aat gca tca gaa aca tgg att ggt ttg agc agc aat aaa att cca gtt      1344
   Asn Ala Ser Glu Thr Trp Ile Gly Leu Ser Ser Asn Lys Ile Pro Val
           435                 440                 445
   tcc ttt gaa tgg tct aat gac tct tca gtc atc ttt act aat tgg cac      1392
   Ser Phe Glu Trp Ser Asn Asp Ser Ser Val Ile Phe Thr Asn Trp His
       450                 455                 460
   aca ctt gag ccc cac att ttt cca aat aga agc cag ctg tgt gtc tca      1440
   Thr Leu Glu Pro His Ile Phe Pro Asn Arg Ser Gln Leu Cys Val Ser
   465                 470                 475                 480
   gca gag cag tct gag gga cac tgg aaa gtc aaa aat tgt gaa gaa aga      1488
   Ala Glu Gln Ser Glu Gly His Trp Lys Val Lys Asn Cys Glu Glu Arg
                   485                 490                 495
   ctt ttt tac att tgt aaa aaa gca ggc cat gtc ctc tct gat gct gaa      1536
   Leu Phe Tyr Ile Cys Lys Lys Ala Gly His Val Leu Ser Asp Ala Glu
               500                 505                 510
   tca gga tgt caa gag gga tgg gag aga cat ggt gga ttc tgt tac aaa      1584
   Ser Gly Cys Gln Glu Gly Trp Glu Arg His Gly Gly Phe Cys Tyr Lys
           515                 520                 525
   att gac aca gtc ctt cga agc ttt gac caa gct tcc agc ggt tat tac      1632
   Ile Asp Thr Val Leu Arg Ser Phe Asp Gln Ala Ser Ser Gly Tyr Tyr
       530                 535                 540
   tgt cct cct gca ctt gta acc att aca aac agg ttt gaa cag gct ttt      1680
   Cys Pro Pro Ala Leu Val Thr Ile Thr Asn Arg Phe Glu Gln Ala Phe
   545                 550                 555                 560
   att acc agt ttg atc agt agt gtg gta aaa atg aag gac agt tat ttt      1728
   Ile Thr Ser Leu Ile Ser Ser Val Val Lys Met Lys Asp Ser Tyr Phe
                   565                 570                 575
   tgg ata gct ctt cag gac caa aat gat acg gga gaa tac act tgg aag      1776
   Trp Ile Ala Leu Gln Asp Gln Asn Asp Thr Gly Glu Tyr Thr Trp Lys
               580                 585                 590
   cca gta ggg cag aaa ccc gag ccg gtg cag tac aca cac tgg aac aca      1824
   Pro Val Gly Gln Lys Pro Glu Pro Val Gln Tyr Thr His Trp Asn Thr
           595                 600                 605
   cac cag ccg cgc tac agt ggt ggc tgt gtt gcc atg cga gga agg cat      1872
   His Gln Pro Arg Tyr Ser Gly Gly Cys Val Ala Met Arg Gly Arg His
       610                 615                 620
   cca ctt ggt cgc tgg gaa gtg aag cac tgt cgg cac ttt aag gca atg      1920
   Pro Leu Gly Arg Trp Glu Val Lys His Cys Arg His Phe Lys Ala Met
   625                 630                 635                 640
   tcc ttg tgc aag cag cca gtt gaa aat cag gaa aaa gca gag tat gaa      1968
   Ser Leu Cys Lys Gln Pro Val Glu Asn Gln Glu Lys Ala Glu Tyr Glu
                   645                 650                 655
   gag aga tgg ccc ttt cac ccc tgc tat ttg gac tgg gag tca gag cct      2016
   Glu Arg Trp Pro Phe His Pro Cys Tyr Leu Asp Trp Glu Ser Glu Pro
               660                 665                 670
   ggt ctg gcc agt tgc ttc aag gta ttt cat agt gaa aaa gtt ctg atg      2064
   Gly Leu Ala Ser Cys Phe Lys Val Phe His Ser Glu Lys Val Leu Met
           675                 680                 685
   aaa aga aca tgg aga gaa gct gaa gca ttt tgc gaa gaa ttt gga gct      2112
   Lys Arg Thr Trp Arg Glu Ala Glu Ala Phe Cys Glu Glu Phe Gly Ala
       690                 695                 700
   cat ctt gca agc ttt gcc cat att gag gaa gag aat ttt gtg aat gag      2160
   His Leu Ala Ser Phe Ala His Ile Glu Glu Glu Asn Phe Val Asn Glu
   705                 710                 715                 720
   ctc tta cat tca aaa ttt aat tgg aca gaa gaa agg cag ttc tgg att      2208
   Leu Leu His Ser Lys Phe Asn Trp Thr Glu Glu Arg Gln Phe Trp Ile
                   725                 730                 735
   gga ttt aat aaa aga aac cca ctg aat gcc ggc tca tgg gag tgg tct      2256
   Gly Phe Asn Lys Arg Asn Pro Leu Asn Ala Gly Ser Trp Glu Trp Ser
               740                 745                 750
   gat aga act cct gtt gtc tct tcg ttt tta gac aac act tat ttt gga      2304
   Asp Arg Thr Pro Val Val Ser Ser Phe Leu Asp Asn Thr Tyr Phe Gly
           755                 760                 765
   gaa gat gca aga aac tgt gct gtt tat aag gca aac aaa aca ttg ctg      2352
   Glu Asp Ala Arg Asn Cys Ala Val Tyr Lys Ala Asn Lys Thr Leu Leu
       770                 775                 780
   ccc tta cac tgt ggt tcc aaa cgt gaa tgg ata tgc aaa atc cca aga      2400
   Pro Leu His Cys Gly Ser Lys Arg Glu Trp Ile Cys Lys Ile Pro Arg
   785                 790                 795                 800
   gat gtg aaa ccc aag att ccg ttc tgg tac cag tac gat gta ccc tgg      2448
   Asp Val Lys Pro Lys Ile Pro Phe Trp Tyr Gln Tyr Asp Val Pro Trp
                   805                 810                 815
   ctc ttt tat cag gat gca gaa tac ctt ttt cat acc ttt gcc tca gaa      2496
   Leu Phe Tyr Gln Asp Ala Glu Tyr Leu Phe His Thr Phe Ala Ser Glu
               820                 825                 830
   tgg ttg aac ttt gag ttt gtc tgt agc tgg ctg cac agt gat ctt ctc      2544
   Trp Leu Asn Phe Glu Phe Val Cys Ser Trp Leu His Ser Asp Leu Leu
           835                 840                 845
   aca att cat tct gca cat gag caa gaa ttc atc cac agc aaa ata aaa      2592
   Thr Ile His Ser Ala His Glu Gln Glu Phe Ile His Ser Lys Ile Lys
       850                 855                 860
   gcg cta tca aag tat ggt gca agt tgg tgg att gga ctt caa gaa gaa      2640
   Ala Leu Ser Lys Tyr Gly Ala Ser Trp Trp Ile Gly Leu Gln Glu Glu
   865                 870                 875                 880
   aga gcc aat gat gaa ttt cgc tgg aga gat gga aca cca gtg ata tac      2688
   Arg Ala Asn Asp Glu Phe Arg Trp Arg Asp Gly Thr Pro Val Ile Tyr
                   885                 890                 895
   cag aac tgg gac aca gga aga gaa aga act gtg aat aat cag agc cag      2736
   Gln Asn Trp Asp Thr Gly Arg Glu Arg Thr Val Asn Asn Gln Ser Gln
               900                 905                 910
   aga tgt ggc ttt att tct tct ata aca gga ctc tgg ggt agt gaa gag      2784
   Arg Cys Gly Phe Ile Ser Ser Ile Thr Gly Leu Trp Gly Ser Glu Glu
           915                 920                 925
   tgt tca gtt tct atg cct agt atc tgt aag cga aaa aag gtt tgg ctc      2832
   Cys Ser Val Ser Met Pro Ser Ile Cys Lys Arg Lys Lys Val Trp Leu
       930                 935                 940
   ata gag aaa aag aaa gat aca cca aaa caa cat gga acg tgt ccc aaa      2880
   Ile Glu Lys Lys Lys Asp Thr Pro Lys Gln His Gly Thr Cys Pro Lys
   945                 950                 955                 960
   gga tgg cta tat ttt aac tat aag tgc ctt ctg ctg aat atc ccc aaa      2928
   Gly Trp Leu Tyr Phe Asn Tyr Lys Cys Leu Leu Leu Asn Ile Pro Lys
                   965                 970                 975
   gac cca agc agt tgg aag aac tgg acg cat gct caa cat ttc tgt gct      2976
   Asp Pro Ser Ser Trp Lys Asn Trp Thr His Ala Gln His Phe Cys Ala
               980                 985                 990
   gaa gaa ggg ggg acc ctg gtc gcc  att gaa agt gag gtg  gag caa gct    3024
   Glu Glu Gly Gly Thr Leu Val Ala  Ile Glu Ser Glu Val  Glu Gln Ala
           995                 1000                1005
   ttc att  act atg aat ctt ttt  ggc cag acc acc agt  gtg tgg ata       3069
   Phe Ile  Thr Met Asn Leu Phe  Gly Gln Thr Thr Ser  Val Trp Ile
       1010                 1015                 1020
   ggt tta  caa aat gat gat tat  gaa aca tgg cta aat  gga aag cct       3114
   Gly Leu  Gln Asn Asp Asp Tyr  Glu Thr Trp Leu Asn  Gly Lys Pro
       1025                 1030                 1035
   gtg gta  tat tct aac tgg tct  cca ttt gat ata ata  aat att cca       3159
   Val Val  Tyr Ser Asn Trp Ser  Pro Phe Asp Ile Ile  Asn Ile Pro
       1040                 1045                 1050
   agt cac  aat acc act gaa gtt  cag aaa cac att cct  ctc tgt gcc       3204
   Ser His  Asn Thr Thr Glu Val  Gln Lys His Ile Pro  Leu Cys Ala
       1055                 1060                 1065
   tta ctc  tca agt aat cct aat  ttt cat ttc act gga  aaa tgg tat       3249
   Leu Leu  Ser Ser Asn Pro Asn  Phe His Phe Thr Gly  Lys Trp Tyr
       1070                1075                1080
   ttt gaa  gac tgt gga aag gaa  ggc tat ggg ttt gtt  tgt gaa aaa      3294
   Phe Glu  Asp Cys Gly Lys Glu  Gly Tyr Gly Phe Val  Cys Glu Lys
       1085                1090                1095
   taa                                                                 3297
<210> 6
   <211> 1098
   <212> PRT
   <213> Homo sapiens
<400> 6
   Met Leu Leu Ser Pro Ser Leu Leu Leu Leu Leu Leu Leu Gly Ala Pro
   1               5                   10                  15
   Arg Gly Cys Ala Glu Gly Val Ala Ala Ala Leu Thr Pro Glu Arg Leu
               20                  25                  30
   Leu Glu Trp Gln Asp Lys Gly Ile Phe Val Ile Gln Ser Glu Ser Leu
           35                  40                  45
   Lys Lys Cys Ile Gln Ala Gly Lys Ser Val Leu Thr Leu Glu Asn Cys
       50                  55                  60
   Lys Gln Ala Asn Lys His Met Leu Trp Lys Trp Val Ser Asn His Gly
   65                  70                  75                  80
   Leu Phe Asn Ile Gly Gly Ser Gly Cys Leu Gly Leu Asn Phe Ser Ala
                   85                  90                  95
   Pro Glu Gln Pro Leu Ser Leu Tyr Glu Cys Asp Ser Thr Leu Val Ser
               100                 105                 110
   Leu Arg Trp Arg Cys Asn Arg Lys Met Ile Thr Gly Pro Leu Gln Tyr
           115                 120                 125
   Ser Val Gln Val Ala His Asp Asn Thr Val Val Ala Ser Arg Lys Tyr
       130                 135                 140
   Ile His Lys Trp Ile Ser Tyr Gly Ser Gly Gly Gly Asp Ile Cys Glu
   145                 150                 155                 160
   Tyr Leu His Lys Asp Leu His Thr Ile Lys Gly Asn Thr His Gly Met
                   165                 170                 175
   Pro Cys Met Phe Pro Phe Gln Tyr Asn His Gln Trp His His Glu Cys
               180                 185                 190
   Thr Arg Glu Gly Arg Glu Asp Asp Leu Leu Trp Cys Ala Thr Thr Ser
           195                 200                 205
   Arg Tyr Glu Arg Asp Glu Lys Trp Gly Phe Cys Pro Asp Pro Thr Ser
       210                 215                 220
   Ala Glu Val Gly Cys Asp Thr Ile Trp Glu Lys Asp Leu Asn Ser His
   225                 230                 235                 240
   Ile Cys Tyr Gln Phe Asn Leu Leu Ser Ser Leu Ser Trp Ser Glu Ala
                   245                 250                 255
   His Ser Ser Cys Gln Met Gln Gly Gly Thr Leu Leu Ser Ile Thr Asp
               260                 265                 270
   Glu Thr Glu Glu Asn Phe Ile Arg Glu His Met Ser Ser Lys Thr Val
           275                 280                 285
   Glu Val Trp Val Gly Leu Asn Gln Leu Asp Glu Asp Ala Gly Trp Gln
       290                 295                 300
   Trp Ser Asp Gly Thr Pro Leu Asn Tyr Leu Asn Trp Ser Pro Glu Val
   305                 310                 315                 320
   Asn Phe Glu Pro Phe Val Glu Asp His Cys Gly Thr Phe Ser Ser Phe
                   325                 330                 335
   Met Pro Ser Ala Trp Arg Ser Arg Asp Cys Glu Ser Thr Leu Pro Tyr
               340                 345                 350
   Ile Cys Lys Lys Tyr Leu Asn His Ile Asp His Glu Ile Val Glu Lys
           355                 360                 365
   Asp Ala Trp Lys Tyr Tyr Ala Thr His Cys Glu Pro Gly Trp Asn Pro
       370                 375                 380
   Tyr Asn Arg Asn Cys Tyr Lys Leu Gln Lys Glu Glu Lys Thr Trp His
   385                 390                 395                 400
   Glu Ala Leu Arg Ser Cys Gln Ala Asp Asn Ser Ala Leu Ile Asp Ile
                   405                 410                 415
   Thr Ser Leu Ala Glu Val Glu Phe Leu Val Thr Leu Leu Gly Asp Glu
               420                 425                 430
   Asn Ala Ser Glu Thr Trp Ile Gly Leu Ser Ser Asn Lys Ile Pro Val
           435                 440                 445
   Ser Phe Glu Trp Ser Asn Asp Ser Ser Val Ile Phe Thr Asn Trp His
       450                 455                 460
   Thr Leu Glu Pro His Ile Phe Pro Asn Arg Ser Gln Leu Cys Val Ser
   465                 470                 475                 480
   Ala Glu Gln Ser Glu Gly His Trp Lys Val Lys Asn Cys Glu Glu Arg
                   485                 490                 495
   Leu Phe Tyr Ile Cys Lys Lys Ala Gly His Val Leu Ser Asp Ala Glu
               500                 505                 510
   Ser Gly Cys Gln Glu Gly Trp Glu Arg His Gly Gly Phe Cys Tyr Lys
           515                 520                 525
   Ile Asp Thr Val Leu Arg Ser Phe Asp Gln Ala Ser Ser Gly Tyr Tyr
       530                 535                 540
   Cys Pro Pro Ala Leu Val Thr Ile Thr Asn Arg Phe Glu Gln Ala Phe
   545                 550                 555                 560
   Ile Thr Ser Leu Ile Ser Ser Val Val Lys Met Lys Asp Ser Tyr Phe
                   565                 570                 575
   Trp Ile Ala Leu Gln Asp Gln Asn Asp Thr Gly Glu Tyr Thr Trp Lys
               580                 585                 590
   Pro Val Gly Gln Lys Pro Glu Pro Val Gln Tyr Thr His Trp Asn Thr
           595                 600                 605
   His Gln Pro Arg Tyr Ser Gly Gly Cys Val Ala Met Arg Gly Arg His
       610                 615                 620
   Pro Leu Gly Arg Trp Glu Val Lys His Cys Arg His Phe Lys Ala Met
   625                 630                 635                 640
   Ser Leu Cys Lys Gln Pro Val Glu Asn Gln Glu Lys Ala Glu Tyr Glu
                   645                 650                 655
   Glu Arg Trp Pro Phe His Pro Cys Tyr Leu Asp Trp Glu Ser Glu Pro
               660                 665                 670
   Gly Leu Ala Ser Cys Phe Lys Val Phe His Ser Glu Lys Val Leu Met
           675                 680                 685
   Lys Arg Thr Trp Arg Glu Ala Glu Ala Phe Cys Glu Glu Phe Gly Ala
   690 695 700
   His Leu Ala Ser Phe Ala His Ile Glu Glu Glu Asn Phe Val Asn Glu
   705 710 715 720
   Leu Leu His Ser Lys Phe Asn Trp Thr Glu Glu Arg Gln Phe Trp Ile
   725 730 735
   Gly Phe Asn Lys Arg Asn Pro Leu Asn Ala Gly Ser Trp Glu Trp Ser
   740 745 750
   Asp Arg Thr Pro Val Val Ser Ser Phe Leu Asp Asn Thr Tyr Phe Gly
   755 760 765
   Glu Asp Ala Arg Asn Cys Ala Val Tyr Lys Ala Asn Lys Thr Leu Leu
   770 775 780
   Pro Leu His Cys Gly Ser Lys Arg Glu Trp Ile Cys Lys Ile Pro Arg
   785 790 795 800
   Asp Val Lys Pro Lys Ile Pro Phe Trp Tyr Gln Tyr Asp Val Pro Trp
   805 810 815
   Leu Phe Tyr Gln Asp Ala Glu Tyr Leu Phe His Thr Phe Ala Ser Glu
   820 825 830
   Trp Leu Asn Phe Glu Phe Val Cys Ser Trp Leu His Ser Asp Leu Leu
   835 840 845
   Thr Ile His Ser Ala His Glu Gln Glu Phe Ile His Ser Lys Ile Lys
   850 855 860
   Ala Leu Ser Lys Tyr Gly Ala Ser Trp Trp Ile Gly Leu Gln Glu Glu
   865 870 875 880
   Arg Ala Asn Asp Glu Phe Arg Trp Arg Asp Gly Thr Pro Val Ile Tyr
   885 890 895
   Gln Asn Trp Asp Thr Gly Arg Glu Arg Thr Val Asn Asn Gln Ser Gln
   900 905 910
   Arg Cys Gly Phe Ile Ser Ser Ile Thr Gly Leu Trp Gly Ser Glu Glu
   915 920 925
   Cys Ser Val Ser Met Pro Ser Ile Cys Lys Arg Lys Lys Val Trp Leu
   930 935 940
   Ile Glu Lys Lys Lys Asp Thr Pro Lys Gln His Gly Thr Cys Pro Lys
   945                 950                 955                 960
   Gly Trp Leu Tyr Phe Asn Tyr Lys Cys Leu Leu Leu Asn Ile Pro Lys
                   965                 970                 975
   Asp Pro Ser Ser Trp Lys Asn Trp Thr His Ala Gln His Phe Cys Ala
               980                 985                 990
   Glu Glu Gly Gly Thr Leu Val Ala  Ile Glu Ser Glu Val  Glu Gln Ala
           995                 1000                 1005
   Phe Ile  Thr Met Asn Leu Phe  Gly Gln Thr Thr Ser  Val Trp Ile
       1010                 1015                 1020
   Gly Leu  Gln Asn Asp Asp Tyr  Glu Thr Trp Leu Asn  Gly Lys Pro
       1025                 1030                 1035
   Val Val  Tyr Ser Asn Trp Ser  Pro Phe Asp Ile Ile  Asn Ile Pro
       1040                 1045                 1050
   Ser His  Asn Thr Thr Glu Val  Gln Lys His Ile Pro  Leu Cys Ala
       1055                 1060                 1065
   Leu Leu  Ser Ser Asn Pro Asn  Phe His Phe Thr Gly  Lys Trp Tyr
       1070                 1075                 1080
   Phe Glu  Asp Cys Gly Lys Glu  Gly Tyr Gly Phe Val  Cys Glu Lys
       1085                 1090                 1095
<210> 7
   <211> 1938
   <212> DNA
   <213> Homo sapiens
<220>
   <221> CDS
   <222> (1)..(1935)
<400> 7
   atg ctg ctg tcg ccg tcg ctg ctg ctg ctg ctg ctg ctg ggg gcg ccg        48
   Met Leu Leu Ser Pro Ser Leu Leu Leu Leu Leu Leu Leu Gly Ala Pro
   1               5                   10                  15
   cgg ggc tgc gcc gag ggt gtg gcg gcg gcg ctt acc ccc gag cgg ctc        96
   Arg Gly Cys Ala Glu Gly Val Ala Ala Ala Leu Thr Pro Glu Arg Leu
               20                  25                  30
   ctg gag tgg cag gat aaa gga ata ttt gtt atc caa agt gag agt ctc       144
   Leu Glu Trp Gln Asp Lys Gly Ile Phe Val Ile Gln Ser Glu Ser Leu
           35                  40                  45
   aag aaa tgc att caa gca ggt aaa tcg gtt ctg acc ctg gag aac tgc      192
   Lys Lys Cys Ile Gln Ala Gly Lys Ser Val Leu Thr Leu Glu Asn Cys
       50                  55                  60
   aag caa gca aac aag cac atg ctg tgg aaa tgg gtt tca aac cat ggc      240
   Lys Gln Ala Asn Lys His Met Leu Trp Lys Trp Val Ser Asn His Gly
   65                  70                  75                  80
   ctc ttt aac ata gga ggc agc ggt tgc ctg ggc ctg aat ttc tcc gcc      288
   Leu Phe Asn Ile Gly Gly Ser Gly Cys Leu Gly Leu Asn Phe Ser Ala
                   85                  90                  95
   cca gag cag cca tta agc tta tat gaa tgt gac tcc acc ctc gtt tcc      336
   Pro Glu Gln Pro Leu Ser Leu Tyr Glu Cys Asp Ser Thr Leu Val Ser
               100                 105                 110
   tta cgg tgg cgc tgt aac agg aag atg atc aca ggc ccg ctg cag tac      384
   Leu Arg Trp Arg Cys Asn Arg Lys Met Ile Thr Gly Pro Leu Gln Tyr
           115                 120                 125
   tct gtc cag gtg gcg cat gac aac aca gtg gtg gcc tca cgg aag tat      432
   Ser Val Gln Val Ala His Asp Asn Thr Val Val Ala Ser Arg Lys Tyr
       130                 135                 140
   att cat aag tgg att tct tat ggg tca ggt ggt gga gac att tgt gaa      480
   Ile His Lys Trp Ile Ser Tyr Gly Ser Gly Gly Gly Asp Ile Cys Glu
   145                 150                 155                 160
   tat cta cac aaa gat ttg cat aca atc aaa ggg aac acc cac ggg atg      528
   Tyr Leu His Lys Asp Leu His Thr Ile Lys Gly Asn Thr His Gly Met
                   165                 170                 175
   ccg tgt atg ttt ccc ttc cag tat aac cat cag tgg cat cat gaa tgt      576
   Pro Cys Met Phe Pro Phe Gln Tyr Asn His Gln Trp His His Glu Cys
               180                 185                 190
   acc cgt gaa ggt cgg gaa gat gac tta ctg tgg tgt gcc acg aca agc      624
   Thr Arg Glu Gly Arg Glu Asp Asp Leu Leu Trp Cys Ala Thr Thr Ser
           195                 200                 205
   cgt tat gaa aga gat gaa aag tgg gga ttt tgc cct gat ccc acc tct      672
   Arg Tyr Glu Arg Asp Glu Lys Trp Gly Phe Cys Pro Asp Pro Thr Ser
       210                 215                 220
   gca gaa gta ggt tgt gat act att tgg gag aag gac ctc aat tca cac      720
   Ala Glu Val Gly Cys Asp Thr Ile Trp Glu Lys Asp Leu Asn Ser His
   225                 230                 235                 240
   att tgc tac cag ttc aac ctg ctt tca tct ctc tct tgg agt gag gca      768
   Ile Cys Tyr Gln Phe Asn Leu Leu Ser Ser Leu Ser Trp Ser Glu Ala
                   245                 250                 255
   cat tct tca tgc cag atg caa gga ggt acg ctg tta agt att aca gat      816
   His Ser Ser Cys Gln Met Gln Gly Gly Thr Leu Leu Ser Ile Thr Asp
               260                 265                 270
   gaa act gaa gaa aat ttc ata agg gag cac atg agc agt aaa aca gtg      864
   Glu Thr Glu Glu Asn Phe Ile Arg Glu His Met Ser Ser Lys Thr Val
           275                 280                 285
   gag gtg tgg gtg ggc ctc aat cag ctg gat gaa gac gct ggc tgg cag      912
   Glu Val Trp Val Gly Leu Asn Gln Leu Asp Glu Asp Ala Gly Trp Gln
       290                 295                 300
   tgg tct gat gga acg ccg ctc aac tat ctg aat tgg agc cca gag gta      960
   Trp Ser Asp Gly Thr Pro Leu Asn Tyr Leu Asn Trp Ser Pro Glu Val
   305                 310                 315                 320
   aat ttt gag cca ttt gtt gaa gat cac tgt gga aca ttt agt tca ttt     1008
   Asn Phe Glu Pro Phe Val Glu Asp His Cys Gly Thr Phe Ser Ser Phe
                   325                 330                 335
   atg cca agt gcc tgg agg agt cgg gat tgt gag tcc acc ttg cca tat     1056
   Met Pro Ser Ala Trp Arg Ser Arg Asp Cys Glu Ser Thr Leu Pro Tyr
               340                 345                 350
   ata tgt aaa aaa tat cta aac cac att gat cat gaa ata gtt gaa aaa     1104
   Ile Cys Lys Lys Tyr Leu Asn His Ile Asp His Glu Ile Val Glu Lys
           355                 360                 365
   gat gcg tgg aaa tat tat gct acc cac tgt gag cct ggc tgg aat ccc     1152
   Asp Ala Trp Lys Tyr Tyr Ala Thr His Cys Glu Pro Gly Trp Asn Pro
       370                 375                 380
   tac aat cgt aat tgc tac aaa ctt cag aaa gaa gaa aag acc tgg cat     1200
   Tyr Asn Arg Asn Cys Tyr Lys Leu Gln Lys Glu Glu Lys Thr Trp His
   385                 390                 395                 400
   gag gct ctg cgt tct tgt cag gct gat aac agt gca tta ata gac ata     1248
   Glu Ala Leu Arg Ser Cys Gln Ala Asp Asn Ser Ala Leu Ile Asp Ile
                   405                 410                 415
   acc tca tta gca gag gtg gag ttt ctt gta acc ctc ctt gga gat gaa     1296
   Thr Ser Leu Ala Glu Val Glu Phe Leu Val Thr Leu Leu Gly Asp Glu
               420                 425                 430
   aat gca tca gaa aca tgg att ggt ttg agc agc aat aaa att cca gtt     1344
   Asn Ala Ser Glu Thr Trp Ile Gly Leu Ser Ser Asn Lys Ile Pro Val
           435                 440                 445
   tcc ttt gaa tgg tct aat gac tct tca gtc atc ttt act aat tgg cac     1392
   Ser Phe Glu Trp Ser Asn Asp Ser Ser Val Ile Phe Thr Asn Trp His
       450                 455                 460
   aca ctt gag ccc cac att ttt cca aat aga agc cag ctg tgt gtc tca     1440
   Thr Leu Glu Pro His Ile Phe Pro Asn Arg Ser Gln Leu Cys Val Ser
   465                 470                 475                 480
   gca gag cag tct gag gga cac tgg aaa gtc aaa aat tgt gaa gaa aga     1488
   Ala Glu Gln Ser Glu Gly His Trp Lys Val Lys Asn Cys Glu Glu Arg
                   485                 490                 495
   ctt ttt tac att tgt aaa aaa gca ggc cat gtc ctc tct gat gct gaa     1536
   Leu Phe Tyr Ile Cys Lys Lys Ala Gly His Val Leu Ser Asp Ala Glu
               500                 505                 510
   tca gga tgt caa gag gga tgg gag aga cat ggt gga ttc tgt tac aaa     1584
   Ser Gly Cys Gln Glu Gly Trp Glu Arg His Gly Gly Phe Cys Tyr Lys
           515                 520                 525
   att gac aca gtc ctt cga agc ttt gac caa gct tcc agc ggt tat tac     1632
   Ile Asp Thr Val Leu Arg Ser Phe Asp Gln Ala Ser Ser Gly Tyr Tyr
       530                 535                 540
   tgt cct cct gca ctt gta acc att aca aac agg ttt gaa cag gct ttt     1680
   Cys Pro Pro Ala Leu Val Thr Ile Thr Asn Arg Phe Glu Gln Ala Phe
   545                 550                 555                 560
   att acc agt ttg atc agt agt gtg gta aaa atg aag gac agt tat ttt      1728
   Ile Thr Ser Leu Ile Ser Ser Val Val Lys Met Lys Asp Ser Tyr Phe
                   565                 570                 575
   tgg ata gct ctt cag gac caa aat gat acg gga gaa tac act tgg aag      1776
   Trp Ile Ala Leu Gln Asp Gln Asn Asp Thr Gly Glu Tyr Thr Trp Lys
               580                 585                 590
   cca gta ggg cag aaa ccc gag ccg gtg cag tac aca cac tgg aac aca      1824
   Pro Val Gly Gln Lys Pro Glu Pro Val Gln Tyr Thr His Trp Asn Thr
           595                 600                 605
   cac cag ccg cgc tac agt ggt ggc tgt gtt gcc atg cga gga agg cat      1872
   His Gln Pro Arg Tyr Ser Gly Gly Cys Val Ala Met Arg Gly Arg His
       610                 615                 620
   cca ctt ggt cgc tgg gaa gtg aag cac tgt cgg cac ttt aag gca atg      1920
   Pro Leu Gly Arg Trp Glu Val Lys His Cys Arg His Phe Lys Ala Met
   625                 630                 635                 640
   tcc ttg tgc aag cag taa                                              1938
   Ser Leu Cys Lys Gln
                   645
<210> 8
   <211> 645
   <212> PRT
   <213> Homo sapiens
<400> 8
   Met Leu Leu Ser Pro Ser Leu Leu Leu Leu Leu Leu Leu Gly Ala Pro
   1               5                   10                  15
   Arg Gly Cys Ala Glu Gly Val Ala Ala Ala Leu Thr Pro Glu Arg Leu
               20                  25                  30
   Leu Glu Trp Gln Asp Lys Gly Ile Phe Val Ile Gln Ser Glu Ser Leu
           35                  40                  45
   Lys Lys Cys Ile Gln Ala Gly Lys Ser Val Leu Thr Leu Glu Asn Cys
       50                  55                  60
   Lys Gln Ala Asn Lys His Met Leu Trp Lys Trp Val Ser Asn His Gly
   65                  70                  75                  80
   Leu Phe Asn Ile Gly Gly Ser Gly Cys Leu Gly Leu Asn Phe Ser Ala
                   85                  90                  95
   Pro Glu Gln Pro Leu Ser Leu Tyr Glu Cys Asp Ser Thr Leu Val Ser
               100                 105                 110
   Leu Arg Trp Arg Cys Asn Arg Lys Met Ile Thr Gly Pro Leu Gln Tyr
           115                 120                 125
   Ser Val Gln Val Ala His Asp Asn Thr Val Val Ala Ser Arg Lys Tyr
       130                 135                 140
   Ile His Lys Trp Ile Ser Tyr Gly Ser Gly Gly Gly Asp Ile Cys Glu
   145                 150                 155                 160
   Tyr Leu His Lys Asp Leu His Thr Ile Lys Gly Asn Thr His Gly Met
                   165                 170                 175
   Pro Cys Met Phe Pro Phe Gln Tyr Asn His Gln Trp His His Glu Cys
               180                 185                 190
   Thr Arg Glu Gly Arg Glu Asp Asp Leu Leu Trp Cys Ala Thr Thr Ser
           195                 200                 205
   Arg Tyr Glu Arg Asp Glu Lys Trp Gly Phe Cys Pro Asp Pro Thr Ser
       210                 215                 220
   Ala Glu Val Gly Cys Asp Thr Ile Trp Glu Lys Asp Leu Asn Ser His
   225                 230                 235                 240
   Ile Cys Tyr Gln Phe Asn Leu Leu Ser Ser Leu Ser Trp Ser Glu Ala
                   245                 250                 255
   His Ser Ser Cys Gln Met Gln Gly Gly Thr Leu Leu Ser Ile Thr Asp
               260                 265                 270
   Glu Thr Glu Glu Asn Phe Ile Arg Glu His Met Ser Ser Lys Thr Val
           275                 280                 285
   Glu Val Trp Val Gly Leu Asn Gln Leu Asp Glu Asp Ala Gly Trp Gln
       290                 295                 300
   Trp Ser Asp Gly Thr Pro Leu Asn Tyr Leu Asn Trp Ser Pro Glu Val
   305                 310                 315                 320
   Asn Phe Glu Pro Phe Val Glu Asp His Cys Gly Thr Phe Ser Ser Phe
                   325                 330                 335
   Met Pro Ser Ala Trp Arg Ser Arg Asp Cys Glu Ser Thr Leu Pro Tyr
               340                 345                 350
   Ile Cys Lys Lys Tyr Leu Asn His Ile Asp His Glu Ile Val Glu Lys
           355                 360                 365
   Asp Ala Trp Lys Tyr Tyr Ala Thr His Cys Glu Pro Gly Trp Asn Pro
       370                 375                 380
   Tyr Asn Arg Asn Cys Tyr Lys Leu Gln Lys Glu Glu Lys Thr Trp His
   385                 390                 395                 400
   Glu Ala Leu Arg Ser Cys Gln Ala Asp Asn Ser Ala Leu Ile Asp Ile
                   405                 410                 415
   Thr Ser Leu Ala Glu Val Glu Phe Leu Val Thr Leu Leu Gly Asp Glu
               420                 425                 430
   Asn Ala Ser Glu Thr Trp Ile Gly Leu Ser Ser Asn Lys Ile Pro Val
           435                 440                 445
   Ser Phe Glu Trp Ser Asn Asp Ser Ser Val Ile Phe Thr Asn Trp His
       450                 455                 460
   Thr Leu Glu Pro His Ile Phe Pro Asn Arg Ser Gln Leu Cys Val Ser
   465                 470                 475                 480
   Ala Glu Gln Ser Glu Gly His Trp Lys Val Lys Asn Cys Glu Glu Arg
                   485                 490                 495
   Leu Phe Tyr Ile Cys Lys Lys Ala Gly His Val Leu Ser Asp Ala Glu
               500                 505                 510
   Ser Gly Cys Gln Glu Gly Trp Glu Arg His Gly Gly Phe Cys Tyr Lys
           515                 520                 525
   Ile Asp Thr Val Leu Arg Ser Phe Asp Gln Ala Ser Ser Gly Tyr Tyr
       530                 535                 540
   Cys Pro Pro Ala Leu Val Thr Ile Thr Asn Arg Phe Glu Gln Ala Phe
   545                 550                 555                 560
   Ile Thr Ser Leu Ile Ser Ser Val Val Lys Met Lys Asp Ser Tyr Phe
                   565                 570                 575
   Trp Ile Ala Leu Gln Asp Gln Asn Asp Thr Gly Glu Tyr Thr Trp Lys
               580                 585                 590
   Pro Val Gly Gln Lys Pro Glu Pro Val Gln Tyr Thr His Trp Asn Thr
           595                 600                 605
   His Gln Pro Arg Tyr Ser Gly Gly Cys Val Ala Met Arg Gly Arg His
       610                 615                 620
   Pro Leu Gly Arg Trp Glu Val Lys His Cys Arg His Phe Lys Ala Met
   625                 630                 635                 640
   Ser Leu Cys Lys Gln
                   645
<210> 9
   <211> 669
   <212> DNA
   <213> Homo sapiens
<220>
   <221> CDS
   <222> (1)..(666)
<400> 9
   atg ctg ctg tcg ccg tcg ctg ctg ctg ctg ctg ctg ctg ggg gcg ccg      48
   Met Leu Leu Ser Pro Ser Leu Leu Leu Leu Leu Leu Leu Gly Ala Pro
   1               5                   10                  15
   cgg ggc tgc gcc gag ggt gtg gcg gcg gcg ctt acc ccc gag cgg ctc      96
   Arg Gly Cys Ala Glu Gly Val Ala Ala Ala Leu Thr Pro Glu Arg Leu
               20                  25                  30
   ctg gag tgg cag gat aaa gga ata ttt gtt atc caa agt gag agt ctc     144
   Leu Glu Trp Gln Asp Lys Gly Ile Phe Val Ile Gln Ser Glu Ser Leu
           35                  40                  45
   aag aaa tgc att caa gca ggt aaa tcg gtt ctg acc ctg gag aac tgc     192
   Lys Lys Cys Ile Gln Ala Gly Lys Ser Val Leu Thr Leu Glu Asn Cys
       50                  55                  60
   aag caa gca aac aag cac atg ctg tgg aaa tgg gtt tca aac cat ggc     240
   Lys Gln Ala Asn Lys His Met Leu Trp Lys Trp Val Ser Asn His Gly
   65                  70                  75                  80
   ctc ttt aac ata gga ggc agc ggt tgc ctg ggc ctg aat ttc tcc gcc     288
   Leu Phe Asn Ile Gly Gly Ser Gly Cys Leu Gly Leu Asn Phe Ser Ala
                   85                  90                  95
   cca gag cag cca tta agc tta tat gaa tgt gac tcc acc ctc gtt tcc     336
   Pro Glu Gln Pro Leu Ser Leu Tyr Glu Cys Asp Ser Thr Leu Val Ser
               100                 105                 110
   tta cgg tgg cgc tgt aac agg aag atg atc aca ggc ccg ctg cag tac     384
   Leu Arg Trp Arg Cys Asn Arg Lys Met Ile Thr Gly Pro Leu Gln Tyr
           115                 120                 125
   tct gtc cag gtg gcg cat gac aac aca gtg gtg gcc tca cgg aag tat     432
   Ser Val Gln Val Ala His Asp Asn Thr Val Val Ala Ser Arg Lys Tyr
       130                 135                 140
   att cat aag tgg att tct tat ggg tca ggt ggt gga gac att tgt gaa     480
   Ile His Lys Trp Ile Ser Tyr Gly Ser Gly Gly Gly Asp Ile Cys Glu
   145                 150                 155                 160
   tat cta cac aaa gat ttg cat aca atc aaa ggg aac acc cac ggg atg     528
   Tyr Leu His Lys Asp Leu His Thr Ile Lys Gly Asn Thr His Gly Met
                   165                 170                 175
   ccg tgt atg ttt ccc ttc cag tat aac cat cag tgg cat cat gaa tgt     576
   Pro Cys Met Phe Pro Phe Gln Tyr Asn His Gln Trp His His Glu Cys
               180                 185                 190
   acc cgt gaa ggt cgg gaa gat gac tta ctg tgg tgt gcc acg aca agc     624
   Thr Arg Glu Gly Arg Glu Asp Asp Leu Leu Trp Cys Ala Thr Thr Ser
           195                 200                 205
   cgt tat gaa aga gat gaa aag tgg gga ttt tgc cct gat ccc taa         669
   Arg Tyr Glu Arg Asp Glu Lys Trp Gly Phe Cys Pro Asp Pro
       210                 215                 220
<210> 10
   <211> 222
   <212> PRT
   <213> Homo sapiens
<400> 10
   Met Leu Leu Ser Pro Ser Leu Leu Leu Leu Leu Leu Leu Gly Ala Pro
   1               5                   10                  15
   Arg Gly Cys Ala Glu Gly Val Ala Ala Ala Leu Thr Pro Glu Arg Leu
               20                  25                  30
   Leu Glu Trp Gln Asp Lys Gly Ile Phe Val Ile Gln Ser Glu Ser Leu
           35                  40                  45
   Lys Lys Cys Ile Gln Ala Gly Lys Ser Val Leu Thr Leu Glu Asn Cys
       50                  55                  60
   Lys Gln Ala Asn Lys His Met Leu Trp Lys Trp Val Ser Asn His Gly
   65                  70                  75                  80
   Leu Phe Asn Ile Gly Gly Ser Gly Cys Leu Gly Leu Asn Phe Ser Ala
                   85                  90                  95
   Pro Glu Gln Pro Leu Ser Leu Tyr Glu Cys Asp Ser Thr Leu Val Ser
               100                 105                 110
   Leu Arg Trp Arg Cys Asn Arg Lys Met Ile Thr Gly Pro Leu Gln Tyr
           115                 120                 125
   Ser Val Gln Val Ala His Asp Asn Thr Val Val Ala Ser Arg Lys Tyr
       130                 135                 140
   Ile His Lys Trp Ile Ser Tyr Gly Ser Gly Gly Gly Asp Ile Cys Glu
   145                 150                 155                 160
   Tyr Leu His Lys Asp Leu His Thr Ile Lys Gly Asn Thr His Gly Met
                   165                 170                 175
   Pro Cys Met Phe Pro Phe Gln Tyr Asn His Gln Trp His His Glu Cys
               180                 185                 190
   Thr Arg Glu Gly Arg Glu Asp Asp Leu Leu Trp Cys Ala Thr Thr Ser
           195                 200                 205
   Arg Tyr Glu Arg Asp Glu Lys Trp Gly Phe Cys Pro Asp Pro
       210                 215                 220
<210> 11
   <211> 34
   <212> DNA
   <213> Artificial
<220>
   <223> Forward primer
<400> 11
   tactcgagcc accatgctgc tgtcgccgtc gctg 34
<210> 12
   <211> 34
   <212> DNA
   <213> Artificial
<220>
   <223> WT reverse primer
<400> 12
   tagcggccgc ttattggtca ctcttctcaa gatc 34
<210> 13
   <211> 33
   <212> DNA
   <213> Artificial
<220>
   <223> FNII reverse primer
<400> 13
   tagcggccgc ttagggatca gggcaaaatc ccc 33
<210> 14
   <211> 34
   <212> DNA
   <213> Artificial
<220>
   <223> CTLD3 reverse primer
<400> 14
   tagcggccgc ttactgcttg cacaaggaca ttgc 34
<210> 15
   <211> 40
   <212> DNA
   <213> Artificial
<220>
   <223> CTLD6 reverse primer
<400> 15
   tagcggccgc ttatttttca caaacaaacc catagccttc 40
<210> 16
   <211> 43
   <212> DNA
   <213> Artificial
<220>
   <223> CTLD8 reverse primer
<400> 16
   tagcggccgc tcacttactg ttaccatcaa attgagcatt caa 43

## Description

Cancer incidence increases because of the aging population and of changes in the life style in developing countries. More than 10 million cancers are detected worldwide each year and about 7 million people died of cancer. Cancers are now representing about 13% of all deaths each year. Breast cancer is the most frequent cancer for women in western countries and still represents about 13% of cancer death for women (and 6% with male included). Pancreatic and kidney cancers are less frequent. But their incidence in term of mortality is high. Indeed, except surgery that can be performed only in the early stages of the disease (most cancers being detected at late stages), the other treatments are frequently inefficient and most of the time do not increase significantly the patient survival. Therefore, there is still a need for new and efficient anti-cancer treatments in particular for treating adenocarcinomas such as breast cancer, pancreatic cancer and kidney cancer.

Cancer cells derive from normal cells that accumulate genetic and epigenetic alterations. During this process and among other steps, normal cells acquire immortality and enhanced stress resistance (Hanahan and Weinberg, 2000). Restoring genetic events lost during carcinogenesis process may thus exert anti-tumoral activity.

US2011/097732 discloses a method for determining the prognosis of breast cancer in a patient by measuring the amount of Wrap53 in the nucleus of cells in tissue sample from said patient.

PLA2R1 (Phospholipase A2 receptor 1) is a large glycosylated membrane protein of 180 kDa which is also known as the M-type sPLA2 receptor. It belongs to the superfamily of C-type lectins, and its closest paralogs are the macrophage mannose receptor, the endo-180/UPARAP protein and the DEC-205 (Engelholm et al., 2009; Shrimpton et al., 2009; Taylor et al., 2005). PLA2R1 is a type I receptor comprising a single transmembrane domain, a short cytoplasmic tail, and a very large extracellular region made up of an N-terminal cysteine-rich domain, a fibronectin-like type II domain, and a tandem repeat of eight distinct C-type lectin-like carbohydrate recognition domains (CTLDs). It also exists as a soluble secreted receptor comprising the full extracellular region. PLA2R1 is expressed in several tissues including lung, kidney, spleen and colon. It was originally discovered two decades ago in skeletal muscle cells (M-type receptor) by virtue of binding of various venom and mammalian secreted phospholipases A2 (sPLA2s) venom. Since then, PLA2R1 has been proposed to mediate several in vitro functions of sPLA2s and to play a pro-inflammatory role in a mouse model of septic shock. However, PLA2R1 is likely a multifunctional receptor for which the multiple set of in vivo biological functions and the diversity of endogenous ligands still remain largely unknown (Lambeau and Lazdunski, 1999; Murakami et al., 2010).

Cellular senescence results in an irreversible proliferation arrest. Concomitantly, specific markers appear in senescent cells. This cellular response may be involved in various physiopathological conditions especially during aging and aging-related diseases (Campisi, 2011; Rodier and Campisi, 2011). Cellular senescence, by inducing a proliferation arrest is proposed to exert anti-tumoral effect at early stage of tumoral development and during anti-tumoral treatment (Braig et al., 2005; Chen et al., 2005; Collado et al., 2005; Ewald et al., 2010).

We have previously shown that a down-regulation of PLA2R1 favors cellular senescence escape whereas its ectopic expression in normal cells induces cellular senescence (Augert et al., 2009). However, any effect of PLA2R1 on cancer cell lines was neither described nor suggested.

Patel et al. (Clin. Cancer Res. 2008, 14(24):8070-8079) describes that cytosolic phospholipase A2-_{α} (cPLA2-_{α}) expression and activation is increased in androgen insensitive cancer cell lines and tissue. Inhibition of cPLA2-_{α} results in cells and xenograft tumor growth inhibition and serves therefore as a potentially effective therapy for hormone refractory prostate cancer. Growth promoting effect of secretory sPLA2-IIA, one of the secretory PLA2s, seems to be via cPLA2-_{α}.

WO2005/104810 describes putative cancer-associated sequences or genes which have been identified by the use of oncogenic retroviruses whose sequences insert into the genome of a host organism resulting in cancer (see paragraphs [0041]-[0042] of WO2005/104810). This document contains no experimental data or teaching demonstrating that insertion in PLA2R1 sequence is linked to cancer occurrence, demonstrating any therapeutic effect of these sequences or of any putative polypeptide encoded by these sequences.

It is now surprisingly shown that PLA2R1 also induces cell death in various cancer cell lines. Further, PLA2R1 levels decrease in cancer cell lines and PLA2R1 is therefore a biomarker for the detection of cancer.

### Summary

The present invention is related to compositions for use in a method for the treatment of cancer, said compositions comprising a polynucleotide selected in the group consisting of:
- The polynucleotide of SEQ ID NO. 1 or a variant thereof having at least 95% identity with the sequence of SEQ ID No. 1;
- A polynucleotide encoding the human PLA2R1 polypeptide of SEQ ID NO. 2 or encoding a variant thereof having at least 95% identity with the sequence of SEQ ID No. 2,
- A polynucleotide from position 1 to position 4191 of SEQ ID NO.1 encoding the extracellular domain of human PLA2R1 or a variant thereof having at least 95% identity with the polynucleotide from position 1 to position 4191 of SEQ ID NO.1;
- A fragment of the polynucleotide from position 1 to position 4191 of SEQ ID NO.1 encoding a fragment of the extracellular domain of PLA2R1 or a variant thereof having at least 95% identity with a fragment of the polynucleotide from position 1 to position 4191 of SEQ ID NO.1, said fragment encoding at least the Cys-rich domain, the fibronectin type II domain and a C-type lectin domain of human PLA2R1;
- A polynucleotide encoding a polypeptide of SEQ ID NO. 4, 6 or 8

More preferably, the fragment of the polynucleotide from position 1 to position 4191 of SEQ ID NO.1 is selected in the group consisting of the polynucleotides of SEQ ID NO. 3, SEQ ID NO. 5, SEQ ID NO. 7 and variants thereof having at least 95% identity with the polynucleotide of SEQ ID NO. 3, SEQ ID NO.5 or SEQ ID NO.7.

In a first embodiment the polynucleotide is inserted in a viral vector. In another embodiment, the polynucleotide is incorporated in a delivery vehicle.

Another object of the present invention is a composition for use in a method for the treatment of cancer, said composition comprising a polypeptide selected in the group consisting of:
- The Human PLA2R1 polypeptide depicted in SEQ ID NO. 2 or a variant thereof having at least 95% identity with the polypeptide of SEQ ID No. 2;
- The extracellular domain of human PLA2R1 polypeptide from position 21 to position 1397 of SEQ ID NO.2 or a variant thereof having at least 95 % identity with the human PLA2R1 polypeptide from position 21 to position 1397 of SEQ ID NO.2;
- A fragment of the extracellular domain of human PLA2R1 polypeptide from position 21 to position 1397 of SEQ ID NO.2 or a variant thereof having at least 95 % identity with a fragment the human PLA2R1 polypeptide from position 21 to position 1397 of SEQ ID NO.2, said fragment comprising at least the Cys-rich domain, the fibronectin type II domain and a C-type lectin domain of human PLA2R1

More preferably, the fragment of the extracellular domain of human PLA2R1 polypeptide from position 21 to position 1397 of SEQ ID NO.2 is selected in the group consisting of the polypeptide from position 21 to position 1322 of SEQ ID NO. 4, the polypeptide from position 21 to position 1098 of SEQ ID NO. 6, the polypeptide from position 21 to position 645 of SEQ ID NO. 7 and variants thereof having at least 95% identity with the polypeptide from position 21 to position 1322 of SEQ ID NO. 4, at least 95% identity with the polypeptide from position 21 to position 1098 of SEQ ID NO. 6 or at least 95% identity with the polypeptide from position 21 to position 645 of SEQ ID NO. 8.

In preferred embodiments, the compositions of the present invention are for use in a method for the treatment of breast cancer, pancreatic cancer, kidney cancer, colorectal cancer or melanoma.

Another object of the present invention is a polypeptide encoding a soluble extracellular fragment of human PLA2R1 consisting of the polypeptide of SEQ ID NOs. 4, 6 or 8.

Another object of the present invention is said polypeptide for use in a method for the treatment of cancer more particularly for use in a method for the treatment of breast cancer, kidney cancer, pancreatic cancer, colorectal cancer and/or melanoma.

Another object of the present invention is a polynucleotide encoding said polypeptide. Preferably, the polynucleotides according to the present invention are for use in a method for the treatment of cancer, more particularly for use in the treatment of breast cancer, kidney cancer, pancreatic cancer, colorectal cancer and/or melanoma.

The present invention also discloses a method for detecting a cancer in a patient comprising the following steps:
- Measuring the level of expression of the PLA2R1 gene in a sample previously taken from a patient,
- Comparing the expression level of the PLA2R1 gene with the expression level of the PLA2R1 gene in a control sample,
wherein a decrease of the PLA2R1 expression level in the sample previously taken from the patient compared to the level of expression of the PLA2R1 gene in the control sample is significant of the presence of a cancer.

Preferably, the method is for detecting breast cancer or a kidney cancer.

Another object of the present invention is a method for determining the prognosis of a cancer in a patient comprising the following steps:
a) Measuring the level of expression of the PLA2R1 gene in cancer cells from a tumour sample previously taken from said patient,
b) Classifying the cancer as having a poor prognosis if the PLA2R1 gene is under-expressed in said cancer cells compared to a control sample.

Preferably, the method is for detecting breast cancer, kidney cancer, pancreatic cancer, colorectal cancer or melanoma. More preferably, the method is for detecting breast cancer or a kidney cancer.

### Sequence listing

SEQ ID No.1: PLA2R1 nucleotide sequence
SEQ ID No.2: PLA2R1 amino acid sequence
SEQ ID No.3: PLA2R1 CysR-CTLD8 nucleotide sequence
SEQ ID No.4: PLA2R1 CysR-CTLD8 amino acid sequence
SEQ ID No.5: PLA2R1 CysR-CTLD6 nucleotide sequence
SEQ ID No.6: PLA2R1 CysR-CTLD6 amino acid sequence
SEQ ID No.7: PLA2R1 CysR-CTLD3 nucleotide sequence
SEQ ID No.8: PLA2R1 CysR-CTLD3 amino acid sequence
SEQ ID No.9: PLA2R1 CysR-FNIIs nucleotide sequence
SEQ ID No.10: PLA2R1 CysR-FNIIs amino acid sequence
SEQ ID No.11: Forward primer
SEQ ID No.12: WT reverse primer
SEQ ID No.13: FNII reverse primer
SEQ ID No.14: CTLD3 reverse primer
SEQ ID No.15: CTLD6 reverse primer
SEQ ID No. 16: CTLD8 reverse primer

### Detailed description of the invention

The present invention describes to polynucleotides and polypeptides derived from human PLA2R1 (Phospholipase A2 receptor 1) and their use in methods for the treatment of cancer in particular for the treatment of breast cancer, kidney cancer, pancreatic cancer, colorectal cancer and melanoma. It has been surprisingly found that expression of polynucleotides encoding PLA2R1 in tumor cells triggers cell death in these tumor cells.

The term "polynucleotide" refers to a single strand nucleotide chain or its complementary strand which can be of the DNA or RNA type, or a double strand nucleotide chain which can be of the cDNA (complementary) or genomic DNA type. The term "polynucleotide" also refers to modified polynucleotides.

The polynucleotides of this invention are isolated or purified from their natural environment. Preferably, the polynucleotides of this invention can be prepared using conventional molecular biology techniques such as those described by Sambrook et al. (Molecular Cloning : A Laboratory Manual, 1989) or by chemical synthesis.

In a first embodiment, the present invention relates to the polynucleotide of SEQ ID NO. 1 or variants thereof having at least 95% identity with the sequence of SEQ ID No.1 for use in the treatment of cancer. The polynucleotide of SEQ ID NO. 1 encodes the human PLA2R1 (phospholipase A2 receptor 1) polypeptide.

The polynucleotide of SEQ ID NO. 1 encodes the different domains of the PLA2R1 polypeptide, the positions on the sequence of SEQ ID NO. 1 are as follows:
- nucleotides 1-60 signal peptide,
- nucleotides 112-483 Cysteine rich domain (CysR),
- nucleotides 517-663 Fibronectin type II domain (FNII),
- nucleotides 712-1065 C-type lectin 1 domain (CTLD1),
- nucleotides 1153-1506 C-type lectin 2 domain (CTLD2),
- nucleotides 1564-1929 C-type lectin 3 domain (CTLD3),
- nucleotides 2017-2391 C-type lectin 4 domain (CTLD4),
- nucleotides 2455-2814 C-type lectin 5 domain (CTLD5),
- nucleotides 2893-3288 C-type lectin 6 domain (CTLD6),
- nucleotides 3361-3696 C-type lectin 7 domain (CTLD7),
- nucleotides 3769-4134 C-type lectin 8 domain (CTLD8),
- nucleotides 4192-4254 transmembrane domain,
- nucleotides 4255-4389 intracellular domain.

Further, the nucleotides from position 61 to 4191 encode the extracellular domain of the mature protein after cleavage of the signal peptide.

The invention is also related to a polynucleotide encoding the human PLA2R1 polypeptide of SEQ ID NO. 2 or a variant thereof having at least 95% identity with the sequence of SEQ ID No. 2 for use in a method for the treatment of cancer.

It has also been found that the expression of the soluble extracellular domain of PLA2R1 or of a fragment thereof in tumour cells triggers cell death in these cells.

In a preferred embodiment, the present invention relates to a polynucleotide from position 1 to position 4191 of SEQ ID NO.1 encoding the extracellular domain of human PLA2R1 or a variant thereof having at least 95% identity with the polynucleotide from position 1 to position 4191 of SEQ ID NO. 1 for use in a method for the treatment of cancer. In another preferred embodiment, the present invention is related to a fragment of the polynucleotide from position 1 to position 4191 of SEQ ID NO.1 encoding a fragment of the extracellular domain of PLA2R1 or a variant thereof having at least 95% identity with a fragment of the polynucleotide from position 1 to position 4191 of SEQ ID NO.1, said fragment encoding at least the Cys-rich domain, the fibronectin type II domain and a C-type lectin domain of human PLA2R1 for use in a method for the treatment of cancer. The positions of these domains on the sequence of SEQ ID NO. 1 are indicated above.

In preferred embodiments, the fragment of the polynucleotide from position 1 to position 4191 of SEQ ID NO.1 is selected in the group consisting of the polynucleotides of SEQ ID NO. 3, SEQ ID NO. 5, SEQ ID NO. 7 and variants thereof having at least 95% identity with the polynucleotide of SEQ ID NO. 3, at least 95% identity with the polynucleotide of SEQ ID NO.5 or at least 95% identity with the polynucleotide of SEQ ID NO.7.

The polynucleotide of SEQ ID NO. 3 corresponds to positions 1-3966 of SEQ ID NO. 1. This polynucleotide is a fragment of the extracellular domain of PLA2R1 and it comprises the sequences encoding the signal peptide, the Cys R domain, the FNII domain and the CTLD domains 1-8 as described above.

The polynucleotide of SEQ ID No. 5 corresponds to positions 1-3294 of SEQ ID NO. 1. This polynucleotide is a fragment of the extracellular domain of PLA2R1 and it comprises the sequences encoding the signal peptide, the Cys R domain, the FNII domain and the CTLD domains 1-6 as described above.

The polynucleotide of SEQ ID No. 7 corresponds to positions 1-1935 of SEQ ID NO. 1. This polynucleotide is a fragment of the extracellular domain of PLA2R1 and it comprises the sequences encoding the signal peptide, the Cys R domain, the FNII domain and the CTLD domains 1-3 as described above.

The present invention is also directed to polynucleotides encoding the extracellular domain of human PLA2R1 of SEQ ID NO. 1 or fragments thereof encoding at least the Cys-rich domain, the fibronectin type II domain and a C-type lectin domain of human PLA2R1, in particular to polynucleotides encoding the polypeptides of SEQ ID NOs. 4, 6 or 8.

The invention also relates to variants of the polynucleotides described above wherein said variants present a degree of identity with the polynucleotides described above. Preferably, these variants are naturally occurring variants found in the human population.

The invention thus relates to variants of the polynucleotides described above presenting at least 95%, 98% and preferably at least 99% identity with these polynucleotides. The term identical polynucleotides refers to nucleotides with no variation or changes between two sequences. These polynucleotides can have a deletion, addition or substitution of at least one nucleotide with respect to the reference polynucleotide. The variants of the polynucleotides described above present at least 95%, 98% and preferably at least 99% identity with these polynucleotides over their whole length.

Preferably, the variants of the polynucleotides described above retain the properties of the polynucleotide from which they are derived. In particular, the polynucleotides of the present invention trigger cell death in tumor cells when they are expressed in these cells.

The term polynucleotide "fragments" refers to a polynucleotide including part but not all of the polynucleotide from which it is derived. The fragments according to this invention retain the properties of the polynucleotide from which they are derived. In particular, the polynucleotides of the present invention trigger cell death in tumor cells when they are expressed in these cells.

The degree of identity between two sequences, quantified by a score, is based on the percentage of identities and/or changes in the sequences. The methods for measuring and identifying the degree of identity between nucleic acid sequences are well known to the man skilled in the art. For example, vectors NTi Vector NTi 9.1.0, alignment program AlignX (Clustal W algorithm) (Invitrogen INFORMAX) can be used. Preferably, the default parameters are used.

The invention also discloses polynucleotides capable of selective hybridization with the polynucleotides described above. Preferably, these polynucleotides retain the properties of the polynucleotide to which they hybridize. In particular, the polynucleotides of the present invention trigger cell death in tumor cells when they are expressed in these cells.

Preferably, selective hybridization is carried out under average conditions of stringency and even more preferably under strict conditions of stringency. The term "sequence capable of selective hybridization" refers to sequences which hybridize with the reference sequence at a significantly higher level than background noise. The level of the signal generated by interaction between the sequence capable of selective hybridization and the reference sequence is generally 10 times, preferably 100 times more intense than that for the interaction with other DNA sequences which generate background noise. The strict hybridization conditions leading to selective hybridization are well known to the man skilled in the art. In general, the hybridization and washing temperature is 5°C below the Tm of the reference sequence at a given pH and for a given ionic force. Typically, the hybridization temperature is at least 30°C for a 15 to 50 nucleotide polynucleotide and at least 60°C for a polynucleotide with over 50 nucleotides. As an example, hybridization is carried out in the following buffer: 6X SSC, 50 mM Tris-HCI (pH 7.5), 1 mM EDTA, 0.02% PVP, 0.02% Ficoll, 0.02% BSA, 500 ug/ml of denatured salmon sperm DNA. Washing is carried out, for example, successively at low stringency in 2X SSC, 0.1% SDS buffer, at average stringency in 0.5X SSC, 0.1% SDS buffer and at high stringency in 0.1X SSC, 0.1%SDS. Hybridization can evidently be carried out using other methods well known to the man skilled in the art (see in particular Sambrook et al., Molecular Cloning : A Laboratory Manual, 1989). Preferably, the polynucleotides which hybridize selectively with a reference polynucleotide retain the function of the reference sequence.

In preferred embodiments, the polynucleotides described above are inserted in a viral vector or a pseudoviral vector. Preferably, the vector is suitable to protect and deliver the polynucleotides described above across the cell membrane into the nucleus, to enable their expression in a specific target cell and more particularly in a tumour cell. This viral vector is suitable for targeting and expression in tumor cells for use in the treatment of cancer. Vectors for use in the methods of the present invention are for example retroviruses or adenoviruses.

In other embodiments, the polynucleotides described above are incorporated in a delivery vehicle and more particularly in a non-viral delivery vehicle. To improve the targeting and expression of a polynucleotide in a tumor cell, the polynucleotide must be protected and delivered to the nucleus of the target cell. Any suitable delivery vehicle may be used in the methods of the present invention including liposomes and polymers.

The present invention also relates to polypeptides derived from human PLA2R1 (Phospholipase A2 receptor 1) and their use in a method for the treatment of cancer in particular in a method for the treatment of breast cancer, kidney cancer and pancreatic cancer. The present invention surprisingly shows that the PLA2R1 polypeptide triggers cell death in tumour or cancer cells.

In a first embodiment, the present invention relates to the human PLA2R1 polypeptide depicted in SEQ ID NO. 2 or a variant thereof having at least 95% identity with the polypeptide of SEQ ID No. 2 for use in the treatment of cancer.

The polypeptide of SEQ ID NO. 2 comprises the different domains of the PLA2R1 polypeptide, the positions on the sequence of SEQ ID NO. 2 are as follows:
- amino acids 1-20 signal peptide,
- amino acids 38-161 Cysteine rich domain (CysR),
- amino acids 173-221 Fibronectin type II domain (FNII),
- amino acids 238-355 C-type lectin 1 domain (CTLD1),
- amino acids 385-502 C-type lectin 2 domain (CTLD2),
- amino acids 522-643 C-type lectin 3 domain (CTLD3),
- amino acids 673-797 C-type lectin 4 domain (CTLD4),
- amino acids 819-938 C-type lectin 5 domain (CTLD5),
- amino acids 965-1096 C-type lectin 6 domain (CTLD6),
- amino acids 1121-1232 C-type lectin 7 domain (CTLD7),
- amino acids 1257-1378 C-type lectin 8 domain (CTLD8),
- amino acids 1398-1418 transmembrane domain,
- amino acids 1419-1463 intracellular domain.

Further, the amino acids from position 21 to 1397 correspond to the extracellular domain of the mature protein after clivage of the signal peptide.

The present invention also shows that the extracellular domain of the mature PLA2R1 polypeptide or fragments of the extracellular domain of the mature PLA2R1 polypeptide trigger cell death in cancer cells.

Accordingly, the present invention is also directed to the extracellular domain of human PLA2R1 polypeptide from position 21 to position 1397 of SEQ ID NO.2 or a variant thereof having at least 95 % identity with the human PLA2R1 polypeptide from position 21 to position 1397 of SEQ ID NO.2.

In other embodiments, the invention relates to a fragment of the extracellular domain of human PLA2R1 polypeptide from position 21 to position 1397 of SEQ ID NO.2 or a variant thereof having at least 95 % identity with a fragment the human PLA2R1 polypeptide from position 21 to position 1397 of SEQ ID NO.2, said fragment comprising at least the Cys-rich domain, the fibronectin type II domain and a C-type lectin domain of human PLA2R1.

The positions of these domains on the sequence of SEQ ID NO. 2 are indicated above.

In preferred embodiments, the fragment of the extracellular domain of human PLA2R1 polypeptide from position 21 to position 1397 of SEQ ID NO.2 is selected in the group consisting of the polypeptide from position 21 to position 1322 of SEQ ID NO. 4, the polypeptide from position 21 to position 1098 of SEQ ID NO. 6, the polypeptide from position 21 to position 645 of SEQ ID NO. 8 and variants thereof having at least 95% identity with the polypeptide from position 21 to position 1322 of SEQ ID NO. 4, at least 95% identity with the polypeptide from position 21 to position 1098 of SEQ ID NO. 6 or at least 95% identity with the polypeptide from position 21 to position 645 of SEQ ID NO. 8.

The polypeptide of SEQ ID NO. 4 corresponds to positions 1 to 1322 of SEQ ID NO. 2. This polypeptide is a fragment of the extracellular domain of PLA2R1 and it comprises the the signal peptide, the Cys R domain, the FNII domain and the CTLD domains 1-8 as described above.

The polypeptide of SEQ ID NO. 6 corresponds to positions 1 to 1098 of SEQ ID NO. 2. This polypeptide is a fragment of the extracellular domain of PLA2R1 and it comprises the the signal peptide, the Cys R domain, the FNII domain and the CTLD domains 1-6 as described above.

The polypeptide of SEQ ID NO. 8 corresponds to positions 1 to 645 of SEQ ID NO. 2. This polypeptide is a fragment of the extracellular domain of PLA2R1 and it comprises the the signal peptide, the Cys R domain, the FNII domain and the CTLD domains 1-3 as described above.

The invention also relates to variants of the polypeptides described above wherein said variants present a degree of identity with the polypeptides described above. Preferably, these variants are naturally occurring variants found in the human population.

The invention thus relates to variants of the polypeptides described above presenting at least 95%, 98% and preferably at least 99% identity with these polypeptides. These polypeptides can have a deletion, addition or substitution of at least one amino acid with respect to the reference polypeptide. Preferably, the variants of the polypeptides described above present at least 95%, 98% and preferably at least 99% identity with these polypeptides over their whole length.

The term identical polypeptides refers to polypeptides with no variation or changes between two sequences.

Preferably, the variants of the polypeptides described above retain the properties of the polypeptides from which they are derived. In particular, the polypeptides of the present invention trigger cell death in tumor cells.

The term polypeptide "fragments" refers to a polypeptide including part but not all of the polypetide from which it is derived. The fragments according to this invention retain the properties of the polypeptide from which they are derived. In particular, the polypeptides of the present invention trigger cell death in tumor cells when they are expressed in these cells.

The degree of identity between two sequences, quantified by a score, is based on the percentage of identities and/or changes in the sequences. The methods for measuring and identifying the degree of identity between amino acid sequences are well known to the man skilled in the art. For example, vectors NTi Vector NTi 9.1.0, alignment program AlignX (Clustal W algorithm) (Invitrogen INFORMAX) can be used. Preferably, the default parameters are used.

The polynucleotides and polypeptides described above are for use in methods for the treatment of cancer most preferably for use in methods for the treatment of breast cancer, kidney cancer, pancreatic cancer, colorectal cancer and melanoma.

Further disclosed is a composition for use as a medicament comprising a polynucleotide or a polypeptide as described above.

The present invention is also related to compositions containing a polynucleotide or a polypeptide as described above for use in a method for the treatment of cancer in particular in the treatment of breast cancer, kidney cancer, pancreatic cancer, colorectal cancer and melanoma.

The present invention describes pharmaceutical compositions for use in a method for the treatment of cancer in particular for the treatment of breast cancer, kidney cancer, pancreatic cancer, colorectal cancer and melanoma.

The present invention describes pharmaceutical compositions comprising:
a) an effective amount of a polynucleotide or a polypeptide as described herein, and
b) a pharmaceutically acceptable carrier, which may be inert or physiologically active.

As used herein, "pharmaceutically-acceptable carriers" includes any and all solvents, dispersion media, coatings, antibacterial and antifungal agents, and the like that are physiologically compatible. Examples of suitable carriers, diluents and/or excipients include one or more of water, saline, phosphate buffered saline, dextrose, glycerol, ethanol, and the like, as well as combination thereof. In many cases, it will be preferable to include isotonic agents, such as sugars, polyalcohols, or sodium chloride in the composition. In particular, relevant examples of suitable carrier include: (1) Dulbecco's phosphate buffered saline, pH ∼ 7.4, containing or not containing about 1 mg/ml to 25 mg/ml human serum albumin, (2) 0.9% saline (0.9% w/v sodium chloride (NaCl)), and (3) 5% (w/v) dextrose; and may also contain an antioxidant such as tryptamine and a stabilizing agent such as Tween 20.

The pharmaceutical compositions described by the present invention may also contain a further therapeutic agent for the treatment of cancers.

The compositions described in the present invention may be in a variety of forms. These include for example liquid, semi-solid, and solid dosage forms, but the preferred form depends on the
intended mode of administration and therapeutic application. Typical preferred compositions are in the form of injectable or infusible solutions. The preferred mode of administration is parenteral (e.g. intravenous, intramuscular, intraperinoneal, subcutaneous). In a preferred embodiment, the compositions are administered intravenously as a bolus or by continuous infusion over a period of time. In another preferred embodiment, they are injected by intramuscular, subcutaneous, intra-articular, intrasynovial, intratumoral, peritumoral, intralesional, or perilesional routes, to exert local as well as systemic therapeutic effects.

Sterile compositions for parenteral administration can be prepared by incorporating the polypeptide or the polynucleotide as described in the present invention in the required amount in the appropriate solvent, followed by sterilization by microfiltration. As solvent or vehicle, there may be used water, saline, phosphate buffered saline, dextrose, glycerol, ethanol, and the like, as well as combination thereof. In many cases, it will be preferable to include isotonic agents, such as sugars, polyalcohols, or sodium chloride in the composition.

The polypeptides or polynucleotides as described herein may also be orally administered. As solid compositions for oral administration, tablets, pills, powders (gelatine capsules, sachets) or granules may be used. In these compositions, the active ingredient according to the invention is mixed with one or more inert diluents, such as starch, cellulose, sucrose, lactose or silica, under an argon stream. These compositions may also comprise substances other than diluents, for example one or more lubricants such as magnesium stearate or talc, a coloring, a coating (sugar-coated tablet) or a glaze.

As liquid compositions for oral administration, there may be used pharmaceutically acceptable solutions, suspensions, emulsions, syrups and elixirs containing inert diluents such as water, ethanol, glycerol, vegetable oils or paraffin oil. These compositions may comprise substances other than diluents, for example wetting, sweetening, thickening, flavoring or stabilizing products.

The doses depend on the desired effect, the duration of the treatment and the route of administration used.

The invention is also related to the use of a polypeptide or a polynucleotide as described herein for the manufacture of a medicament for treatment of cancer and more particularly for treatment of breast cancer, kidney cancer, pancreatic cancer, colorectal cancer and melanoma. The present invention also discloses a method for detecting a cancer in a patient comprising the following steps:
- Measuring the level of expression of the PLA2R1 gene in a sample previously taken from a patient,
- Comparing the expression level of the PLA2R1 gene with the expression level of the PLA2R1 gene in a control sample,
wherein a decrease of the PLA2R1 expression level in the sample previously taken from the patient compared to the level of expression of the PLA2R1 gene in the control sample is significant of the presence of a cancer.

Another object of the present invention is a method for determining the prognosis of a cancer in a patient comprising the following steps:
c) Measuring the level of expression of the PLA2R1 gene in cancer cells from a tumour sample previously taken from said patient,
d) Classifying the cancer as having a poor prognosis if the PLA2R1 gene is under-expressed in said cancer cells compared to a control sample.

The present invention also discloses methods for determining the prognosis of a cancer in a patient comprising the following steps:
a) Obtaining a tumour sample from said patient,
b) Measuring the level of expression of the PLA2R1 gene in cancer cells from said tumour sample,
c) Classifying the cancer as having a poor prognosis if the PLA2R1 gene is under-expressed in said cancer cells.

The present invention also relates to methods of determining the prognosis of a patient diagnosed with cancer.

"Cancer" refers to diseases in which a group of cells displays uncontrolled growth/division, invasion and sometimes metastasis.

The methods of the present invention preferably relate to breast cancer, kidney cancer, pancreatic cancer, colorectal cancer and melanoma. In preferred embodiments, the methods of the present invention relate to breast cancer, kidney cancer or pancreatic cancer and even more preferably to breast cancer and kidney cancer.

In a first embodiment, the present invention is related to a method for prognosis of cancer comprising determining the level of expression of PLA2R1 in a biological sample from a human subject. The methods of the present invention are *in vitro* methods. The methods of the present invention are carried out on a biological sample previously taken from a patient. Typically, the patient has been diagnosed with cancer or is suspected of suffering from cancer.

The term "sample" refers to any biological sample obtained/taken from a patient including a blood sample, a plasma sample, a tissue sample, a cell sample or a tumor sample. Typically, the sample contains cancer or tumors cells.

The term "diagnosis" refers both to diagnosis and prognosis of cancer.

The present invention further describes a method for identifying a cancer and/or a tumor having or prone to develop an invasive or metastatic phenotype.

The terms "invasive" or "aggressive" refer to a cancer or to a quickly growing tumor having a tendency to extend beyond its boundaries into adjacent tissues.

The term "metastatic" refers to the spread of a tumor from the organ of origin to additional organs/distal sites in the patient.

A "prognosis" is the likely course and outcome of a disease. The prognosis may include the likelihood of complications of the cancer, of metastasis, of spread, probable outcome of the cancer, likelihood of recovery, overall survival rate and /or overall death rate. Preferably, it is the probability that a patient will recover or have a recurrence/relapse of the cancer. This information is useful to the patient but also to the physician in determining the most effective course of treatment. A determination of the likelihood for a cancer relapse or of the likelihood of metastasis can assist the physician in determining whether a more conservative or a more radical approach to therapy should be taken. Prognosis provides for selection and classification of patients who are predicted to benefit from a given therapeutic regimen.

The methods of the present invention provide prognosis for cancer after it has been diagnosed and/or during therapeutic treatment.

Under-expression or low expression levels of PLA2R1 in cancer cells are characteristic of tumors having a poor prognosis for disease-free survival (DFS) and/or a poor prognosis for overall survival (OS). Under-expression of this gene in cancer cells is statistically significantly correlated with increased disease recurrence and worse prognosis.

The present invention describes a method for identifying a cancer and/or a tumor prone to recur and/or a cancer and/or a tumor having or prone to develop an invasive or metastatic phenotype. More specifically, the present invention describes a method for identifying a cancer and/or a breast tumor prone to recur and/or a cancer and/or a tumor having or prone to develop an invasive or metastatic phenotype.

The term "DFS" refers to Disease Free Survival and is defined as the percentage of patients staying free of disease progression during a period of time. In this case, the Kaplan-Meier curve represents the x % of patients staying free of disease progression after y amount of time.

The DFS in a patient diagnosed with a cancer exhibiting under-expression or low expression levels of PLA2R1 in cancer cells, is reduced compared to a patient who has not an under-expression of PLA2R1 in cancer cells.

The term "OS" refers to Overall Survival and is defined as the percentage of patients who survive after diagnosis of a cancer. In this case, the Kaplan Meier curve represents the x% of patients who survived after y amount of time.

The OS in a patient diagnosed with a cancer exhibiting under-expression or low expression levels of PLA2R1 in cancer cells, is reduced compared to a patient who has not an under-expression of PLA2R1 in cancer cells.

The present invention also discloses a method for diagnosing a cancer in a patient comprising the following steps:
- Measuring the level of expression of the PLA2R1 gene in a sample previously taken from a patient,
- Comparing the expression level of the PLA2R1 gene with the expression level of the PLA2R1 gene in a control sample,
wherein a decrease of the PLA2R1 expression level in the sample previously taken from the patient compared to the level of expression of the PLA2R1 gene in the control sample is significant of an invasive, aggressive or metastatic cancer more particularly of a breast cancer, kidney cancer, pancreatic cancer colorectal cancer or melanoma.

In the methods of the present invention, the level of expression of the PLA2R1 gene is compared to a control sample.

The control may be a "normal" control sample meaning a non-tumor cell control sample or a non-cancerous cell control sample. The control sample can be an autologous control sample obtained from the patient. In that case the sample is obtained from the same patient from which the sample to be evaluated is obtained. The control sample is preferably from the same cell type or from the same organ. The control sample can be a normal control sample obtained from an individual who does not have cancer.

The normal control sample can also be a standard sample that contains the same amount of PLA2R1 that is normally found in biological samples.

In some embodiments, the control sample is the median level of expression of the PLA2R1 gene observed in a healthy population.

In other embodiments, the control sample is the median level of expression of PLA2R1 in samples taken from patients suffering from a cancer and more particularly from breast cancer, kidney cancer, pancreatic cancer colorectal cancer or melanoma.

The term "expression" may refer to measuring the level of transcription and/or the level of translation of the gene.

Preferably, the methods of the present invention comprise measuring the level of expression of the PLA2R1 gene in a sample by quantification of the mRNA of the PLA2R1 gene.

In other embodiments, the methods of the present invention comprise measuring the level of expression of the PLA2R1 gene in a sample by quantification of the polypeptide(s) encoded by the PLA2R1 gene.

Expression levels of the PLA2R1 gene are determined by any of a variety of methods known to the skilled person.

### Figures

Figure 1:
   A/ mRNA were extracted for each cell line. qRT-PCR were performed and relative PLA2R1 mRNA levels were calculated using ACTB, PGK1as housekeeping genes (* P<0, 05; ** P<0, 01; *** P<0, 001).
   B/ mRNA were from 18 normal kidney and 18 tumoral kidney samples were prepared. qRT-PCR were performed and relative PLA2R1 mRNA levels were calculated using ACTB housekeeping gene.
   C/ Cells were infected by a control or a PLA2R1 encoding vector and puromycin selected. Cell extracts were prepared and analyzed by immunoblot for PLA2R1 expression. TUB (tubulin) was used as a loading control.
   D/ Cells seeded at the same density were infected by a control or a PLA2R1 encoding vector, in conditions were all cells were infected. Puromycin was added every day to maintain PLA2R1 expression. Ten days later, cells were PFA-fixed and stained with crystal violet.
   E/ Cells extracts were prepared as in (B) and analyzed by immunoblot for the presence of cleaved caspase-3 (c-CASP3). Actin (ACTB) was used as a loading control.

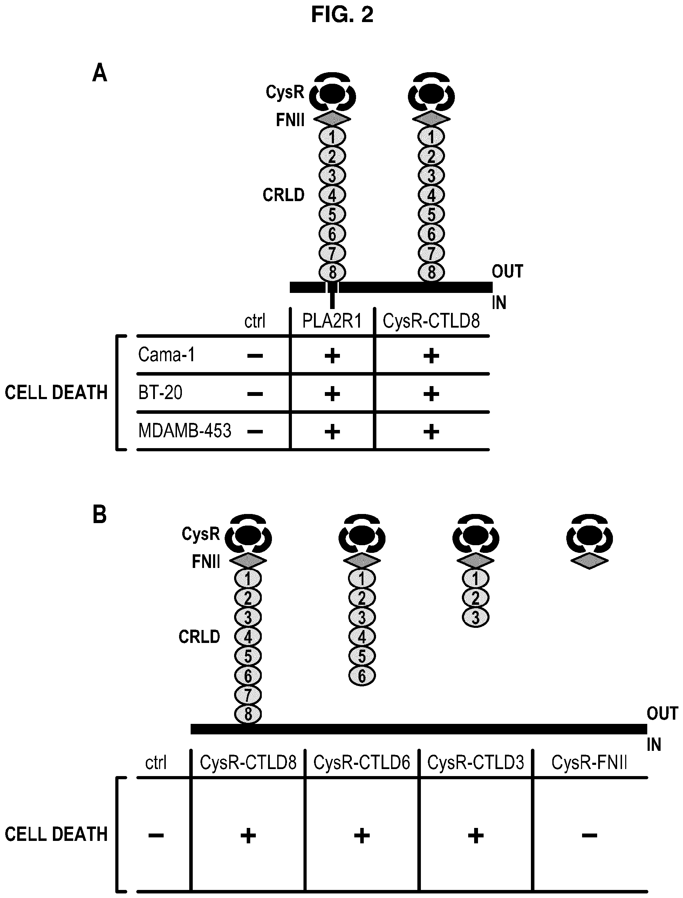

Figure 2: Cells were seeded at the same density, infected by a retrovirus coding for no protein (control), WT PLA2R1 or soluble deletion mutants of PLA2R1, in conditions where all cells were infected. Puromycin was added every day to maintain PLA2R1 expression.
   A/ The effect of control, WT PLA2R1 and CysR-CTLD8 PLA2R1 were assayed in cama-1, BT-20 and MDAMB-453 breast cancer cells for the induction of cell death.
   B/ The effect over cell death of CysR-CTLD8, CysR-CTLD6, CysR-CTLD3 and CysR-FNII soluble forms of PLA2R1 were assayed in Cama-1 breast cancer cells.
Figure 3: Under-expression of PLA2R1 in cancer cells correlates with an increased risk of developing metastasis.
Figure 4: Under-expression of PLA2R1 in cancer cells correlates with decreased Overall Survival (OS).

### Examples

### MATERIALS AND METHODS

### Cell Culture

Human cancer cell lines (ATCC) were cultured in the following media: DMEM (Invitrogen) for MDA-MB 453, MDA-MB-157, MDA-MB-231, MDA-MB-361, MDA-MB-436, Hs-578T, MCF-7, Cal-51, Caki-2, 786-O, RPMI (Invitrogen) for RCC4, RCC10, MEM (Invitrogen) for Cama-1, BT-20, ACHN and Mc Coy's (Invitrogen) for SK-Br3 cells. All medium were supplemented with 10 % FBS (Lonza), 1% penicillin/streptomycin (Invitrogen), 0, 36 % gentamycin (Invitrogen). For Cama-1 and ACHN cells lines, 1% Non essential Amino-Acid (Invitrogen) was added. Virus producing GP293 cells (Clontech) were cultured in DMEM media (Invitrogen) supplemented with 10 % FBS (Lonza), 1% penicillin/streptomycin (Invitrogen) and 0, 36 % gentamycin (Invitrogen).

### Vectors construction

Wild-type membrane-bound (GenBank NM 007366) and soluble deletion mutants of human PLA2R1 were generated by PCR. Each construct was first ligated into the pGEMTeasy vector (Promega), fully sequenced and subsequently subcloned in the pLPCX retroviral vector (Clontech) using XhoI/NotI restriction sites. The PLA2R1 WT (amino acids (aa) 1-1463) and soluble deletion mutants of PLA2R1, namely CysR-FNIIs (aa 1-222), CysR-CTLD3 (aa 1-645) CysR-CTLD6 (aa 1-1098) and CysR-CTLD8 (aa 1-1322) were obtained by using the same forward primer with different reverse primers whose sequences are: Forward, 5'-TACTCGAGCCACCATGCTGCTGTCGCCGTCGCTG-3'; WT Reverse, 5'-TAGCGGCCGCTTATTGGTCACTCTTCTCAAGATC-3'; FNII reverse, 5'-TAGCGGCCGCTTAGGGATCAGGGCAAAATCCCC-3'; CTLD3 reverse, 5'-TAGCGGCCGCTTACTGCTTGCACAAGGACATTGC-3'; CTLD6 reverse, 5'-TAGCGGCCGCTTATTTTTCACAAACAAACCCATAGCCTTC-3'; CTLD8 reverse, 5'-TAGCGGCCGCTCACTTACTGTTACCATCAAATTGAGCATTCAA-3'. PCR conditions were 98°C for 1 minute, followed by 30 cycles of 98°C for 10 seconds, 60°C for 30 seconds, 72°C for 1-5 minutes. This was followed by a final extension of 72°C for 10 minutes.

### Transfection and infection

GP293 cells were transfected using PEI reagents according to manufacturer's recommendations (Euromedex). Two days after transfection, viral supernatant mixed with fresh media (1/2) and polybrene (final concentration at 8ug/mL) was used to infect target cells. One day post infection, cells were selected using puromycin at the final concentration of 0,75-1,5µg/ml depending on the cell type.

### Antibodies and immunoblot

Cell lysates were prepared in ice cold Giordano buffer (50 mM Tris·HCl, pH 7.4, 250 mM NaCl, 0.2% Triton X-100, 5 mM EDTA) supplemented with protease and eroxidise inhibitors (Roche). Lysates were clarified by centrifugation at 14,000 rpm for 30 min at 4°C. Protein concentrations were measured using Bradford protein assay (Biorad #500-0006). For immunoblot analysis, cell extracts were separated by SDS-PAGE gel electrophoresis under non reducing conditions and proteins were transferred onto nitrocellulose membrane. The following primary antibodies were used: anti-PLA2R1 (HPA012657, Atlas), anti-caspase3 (ab32042, Abcam), anti-tubulin (T6199, Sigma) and anti-actin (A5316, Sigma). After incubation with primary antibodies, blots were washed, incubated with a secondary antibody coupled to peroxidase, washed again and antigen-antibody complexes were detected using ECL (Amersham).

### Bioinformatics analysis

The analysis was performed by interrogating the oncomine public database.

### RNA extraction, retro-transcription and quantitative PCR

Normal and tumoral human kidney tissues were provided by the tumorothèque HEH. Total RNA extraction was performed using a phenol-chloroform method using TriReagent (Sigma-Aldrich, Saint Louis, MO, USA). PhaseLockGel tubes (Eppendorf, Hamburg, Germany) were used for phase separation. The synthesis of cDNA was performed from 3ug of total RNA using the First-Strand cDNA Synthesis Kit (GE Healthcare, Chalfont St Giles, UK). The RT reaction was diluted 1/60 and used as cDNA template for qPCR analysis. TaqMan quantitative PCR analysis was carried out on a LightCycler 2.0 System (Roche Applied Science). PCR mixtures contained LightCycler TaqMan mix, 200 nM primers and 1.67µl of cDNA template in a 6.67µl reaction volume. Housekeeping genes (*ACTB, PGK1*), used for normalization of target mRNA expression in each sample type, were selected by systematic geNorm analysis as previously described (Vandesompele et al., 2002). Real-time PCR intron-spanning assays were designed using the ProbeFinder software (Roche Applied Science).

### Colony formation assays

Colony formation assays were carried out in 12-well plates with 100000 cells per well for MDAMB-453 and Cama-1 cells, and 25000 cells plated per well for BT-20 and MDAMB-231 cells. Ten days later, cells were washed with PBS, fixed with 4% paraformaldehyde and stained with 0.05 % Crystal violet (Sigma-Aldrich).

### RESULTS

### PLA2R1 expression is decreased in breast and kidney cancers

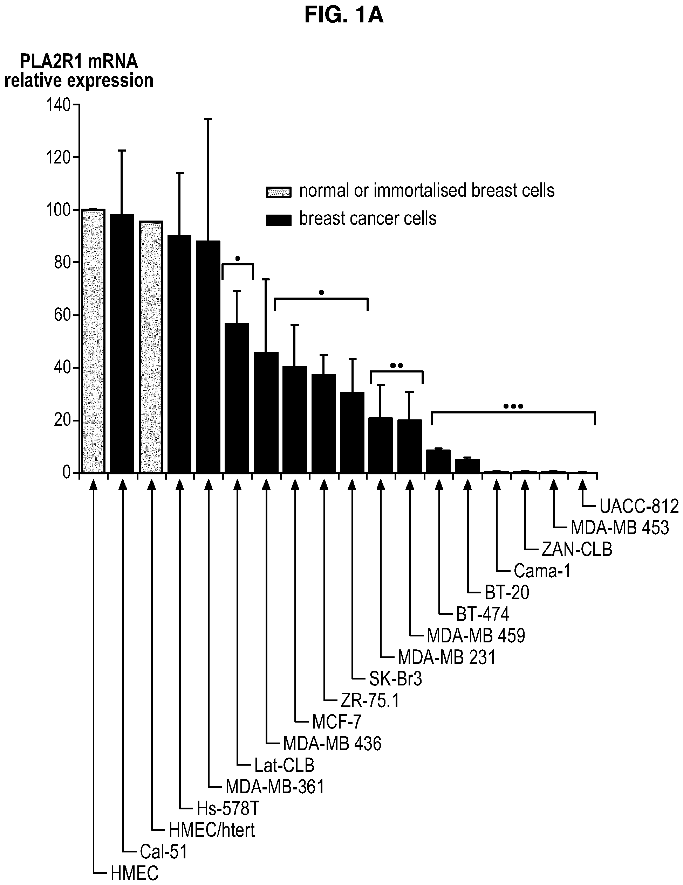

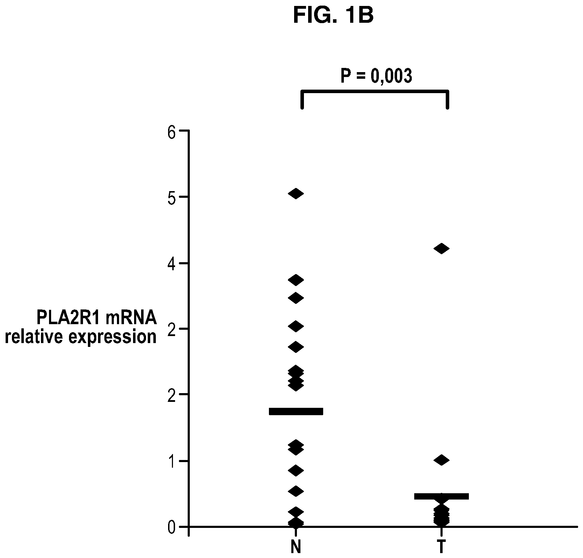

To analyze the expression levels of PLA2R1 in various types of cancer, we performed a bio-informatic analysis of public databases.
The oncomine public database was interrogated for a differential level of PLA2R1 mRNA levels between cancer and normal counterpart in 239 independent set of microarrays analyses. Analysis were performed according to the following criteria: 1/ fold change of PLA2R1 mRNA levels between cancer and normal counterpart, 2/ reproducibility in the differentials between independent studies from the same type of cancer, 3/ fold-change ranking of PLA2R1 in the differentially expressed genes between cancer and normal counterpart and 4/ the p-value. According to these criteria, PLA2R1 mRNA levels were found to significantly decrease in breast and kidney cancers when compared to normal counterpart (Table 1 and 2). This bio-informatics analysis was further confirmed: 1/ by analyzing PLA2R1 mRNA levels in normal or immortalized human breast cells vs human breast cancer cells. Indeed, 13 out of 15 breast cancer cell lines examined displayed a significant decrease in PLA2R1 mRNA levels when compared to normal breast cells (Figure 1A) 2/ by analyzing PLA2R1 mRNA levels in normal vs tumoral human kidney samples (Figure 1B).
Thus a decrease in PLA2R1 expression constitutes a new biomarker of breast and kidney cancers.

**Table 1: Summary of PLA2R1 expression in breast tumors versus normal samples. Six out of 8 studies indicate significant (p<0.05) down-regulation of PLA2R1 in breast tumors**

| Name of the study | Tissues compared | Fold changes | P value | Under-expression Gene Rank |
|---|---|---|---|---|
| Zhao Breast | Invasive Ductal Breast Carcinoma vs. Normal | -2,52 | 1.30E-15 | in top 1% |
| | Lobular Breast Carcinoma vs. Normal | -1,81 | 1.45E-6 | In top 1% |
| Richardson Breast 2 | Ductal Breast Carcinoma vs. Normal | -3.684 | 2.13E-8 | in top 3% |
| Sorlie Breast | Lobular Breast Carcinoma vs. Normal | -1.757 | 0.036 | in top 7% |
| | Ductal Breast Carcinoma vs. Normal | -2.484 | 0.009 | in top 10% |
| Sorlie Breast 2 | Lobular Breast Carcinoma vs. Normal | -1.789 | 0.026 | in top 9% |
| | Ductal Breast Carcinoma vs. Normal | -2.498 | 0.008 | in top 11% |
| Perou Breast | Ductal Breast Carcinoma vs. Normal | -2.411 | 0.043 | in top 15% |
| Turashvili Breast | Invasive Ductal Breast Carcinoma vs. Normal | -2.701 | 0.019 | in top 6% |

**Table 2: Summary of PLA2R1 expression in kidney tumors versus normal samples. Six out of 6 studies indicate significant (p<0.05) down-regulation of PLA2R1 in kidney tumors.**

| Name of the study | Tissues compared | Fold changes | P value | Under-expression Gene Rank |
|---|---|---|---|---|
| Yusenko Renal | Chromophobe Renal Cell Carcinoma vs. Normal | -76.573 | 4.14^{E}-5 | in top 1% |
| | Papillary Renal Cell Carcinoma vs. Normal | -2.111 | 2.94^{E}-8 | in top 1% |
| | Clear Cell Renal Cell Carcinoma vs. Normal | -13.649 | 2.46^{E}-8 | in top 1% |
| Higgins Renal | Granular Renal Cell Carcinoma vs. Normal | -2.714 | 0.031 | in top 6% |
| | Chromophobe Renal Cell Carcinoma vs. Normal | -3.081 | 0.031 | in top 9% |
| | Papillary Renal Cell Carcinoma vs. Normal | -3.202 | 0.028 | in top 13% |
| | Clear Cell Renal Cell Carcinoma vs. Normal | -2.612 | 0.042 | in top 19% |
| Cutcliffe Renal | Renal Wilms Tumor vs. Normal | -4.639 | 4.11^{E}-4 | in top 4% |
| | Clear Cell Sarcoma of the Kidnev vs. Normal | -6.910 | 1.16^{E}-4 | in top 4% |
| Lenburg Renal | Clear Cell Renal Cell Carcinoma vs. Normal | -2.032 | 1.83^{E}-4 | in top 4% |
| Beroukhim Renal | Non-Hereditary Clear Cell Renal Cell Carcinoma vs. Normal | -1.540 | 4.38^{E}-4 | in top 13% |
| | Hereditarv Clear Cell Renal Cell Carcinoma vs. Normal | -1.576 | 2.96^{E}-4 | in top 20% |
| Gumz Renal | Clear Cell Renal Cell Carcinoma vs. Normal | -3.745 | 0.006 | in top 19% |

### PLA2R1 expression induces cancer cell death

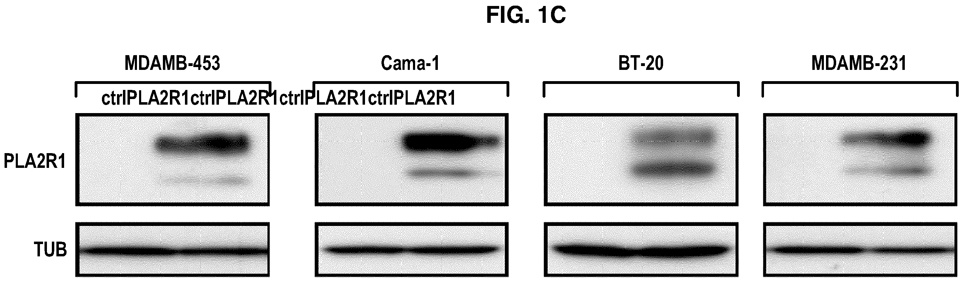

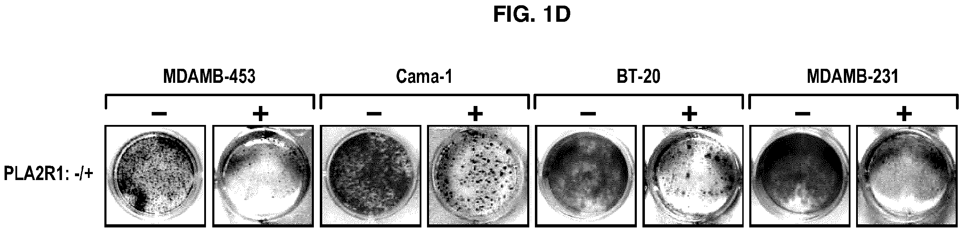

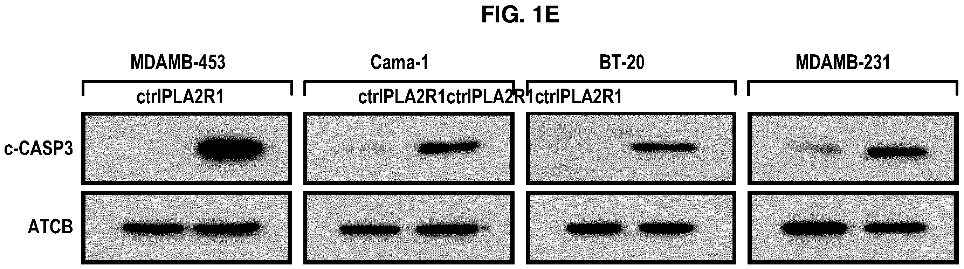

In order to examine the effect of restoring PLA2R1, we have constructed a retroviral vector encoding the full length PLA2R1. PLA2R1 non-expressing cells such as MDAMB-453 and cama-1 breast cancer cells (Figure 1A) were infected by a retroviral vector encoding PLA2R1. Three days post-infection, the cells clearly express PLA2R1 according to the western blot analysis (Figure 1C). Strikingly, PLA2R1 constitutive expression induced a strong growth arrest (Figure 1D) that was due to apoptotic cell death based on the appearance of active cleaved caspase-3 (Figure 1E). Interestingly, cells expressing low levels of PLA2R1 mRNA (BT-20 and MDAMB-231, Figure 1A) were also dying by apoptotic cell death in response to PLA2R1 ectopic expression (Figure 1C-E). In fact, most of the breast cancer cells tested (10 out of 11) died when PLA2R1 was constitutively expressed, without any link with their endogenous mRNA PLA2R1 levels (Table 3). To determine whether the toxic effect of PLA2R1 over-expression is specific to breast cancer cells or more general, we investigated the effect of PLA2R1 ectopic expression in kidney cancer, pancreatic cancer, colorectal cancer and melanoma cells. Five kidney, 2 pancreatic, 1 colorectal cancer and 2 melanoma cell lines were transduced to express PLA2R1 and all of them died in response to PLA2R1 (Table 3). Thus, PLA2R1 constitutive expression induced cell death in almost all (20/21) the cancer cell lines tested.
To determine which PLA2R1 domains are involved in cell death-induced by PLA2R1 ectopic expression, we generated a set of shorter PLA2R1 proteins. A mutant deleted from the intra-cellular domain and of the transmembrane domain (CysR-CTLD8 mutant) was still able to induce apoptotic cell death (Figure 2A). So far, the CysR-CTLD3 mutant, encompassing the cysteine rich domain, the fibronectin-like domain and CTLDs 1 to 3, is the shortest mutant that is still capable of inducing cell death (Figure 2B).
Thus, full-length PLA2R1 and various soluble variants of PLA2R1 are capable of inducing tumor cell death.

**Table 3: Sensitivity (cell death) or resistance (no cell death) to PLA2R1 expression of every human cancer cell lines tested is presented.**

| Name of the cell lines | Sensitive to PLA2R1 | Resistant to PLA2R1 | Tumor origin |
|---|---|---|---|
| MDAMB-453 | X | | Breast |
| Cama-1 | X | | Breast |
| BT-20 | X | | Breast |
| MDAMB-157 | X | | Breast |
| MCF-7 | X | | Breast |
| Cal-51 | X | | Breast |
| MDAMB-436 | X | | Breast |
| MDAMB-231 | X | | Breast |
| MDAMB-361 | X | | Breast |
| Hs-578T | X | | Breast |
| SKBR-3 | | X | Breast |
| Caki-2 | X | | Kidney |
| ACHN | X | | Kidney |
| 786-0 | X | | Kidney |
| RCC4 | X | | Kidney |
| RCC10 | X | | Kidney |
| MIA PaCa-2 | X | | Pancreas |
| Colo 357 | X | | Pancreas |
| A375 | X | | melanoma |
| SK-Mel | X | | melanoma |
| RPMI 7951 | X | | melanoma |
| Lovo | X | | colorectal |
| HT-29 | X | | colorectal |

In conclusion, we have described a new tumor-suppressive-like role of PLA2R1 in cancer. Since PLA2R1 levels decrease in both breast and kidney cancers, PLA2R1 can be useful as a biomarker for these cancer types. In addition, since over-expression of PLA2R1 (full-length or soluble variants) to almost all of the breast, kidney and pancreatic cancer cells induces cell death, PLA2R1 or these shorter mutants can be useful as anti-tumoral biomolecules.

### PLA2R1 expression decrease correlates to an increase risk of developing metastasis and of dying

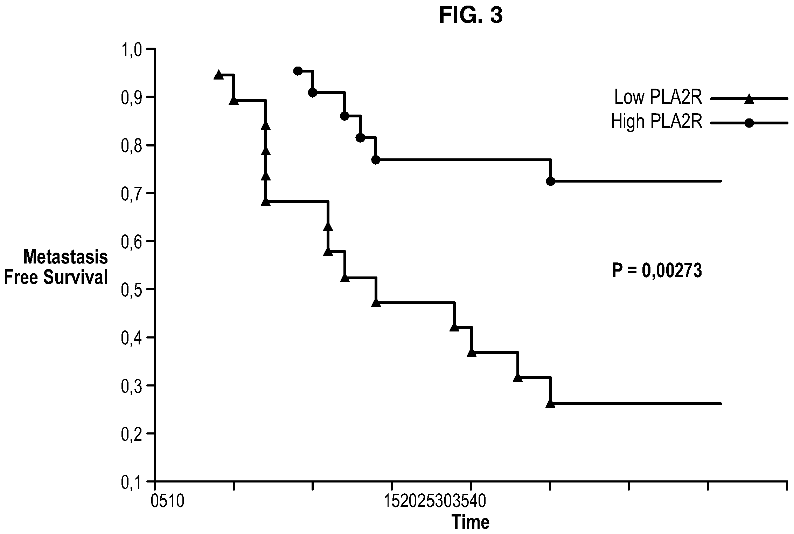

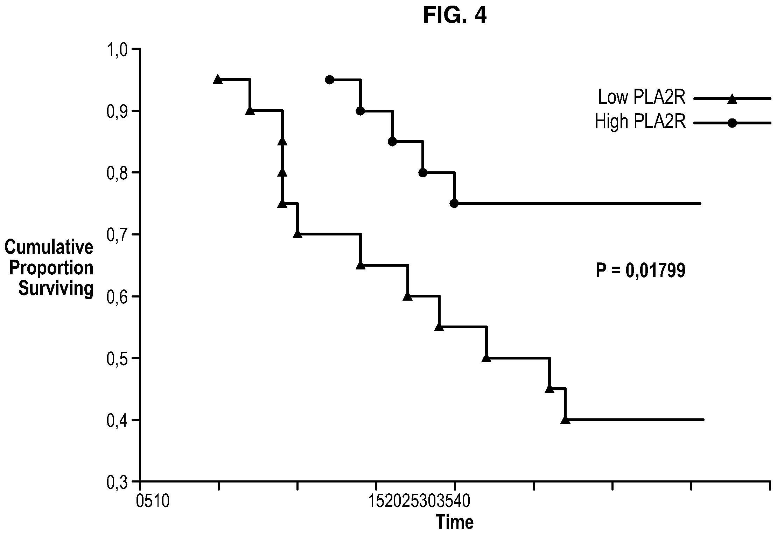

The expression of PLA2R1 decreases in breast and kidney cancer cells. To know whether this decrease might be associated with a clinical behavior, we examined the expression of PLA2R1 on breast primary tumors coming from 20 patients that have developed some metastasis and have died in less than 3 years and from 20 patients that have not developed any metastasis and have not died at 3 years. PLA2R1 mRNA levels were determined by RT-qPCR. A first group containing the half of the samples expressing the higher levels of PLA2R1 and second group containing the half of the samples expressing the lower levels of PLA2R1 was constituted. Survival and metastasis free survival were analyzed in the 2 groups by Kaplan-Meier curves. Interestingly, low level of PLA2R1 was associated with an increase risk of developing metastasis and of dying (see Figures 3 and 4).

### REFERENCES

Augert,A., Payre,C., de Launoit,Y., Gil,J., Lambeau,G., and Bernard,D. (2009). The M-type receptor PLA2R regulates senescence through the p53 pathway. EMBO Rep 10, 271-277.
Braig,M., Lee,S., Loddenkemper,C., Rudolph,C., Peters,A.H., Schlegelberger,B., Stein,H., Dorken,B., Jenuwein,T., and Schmitt,C.A. (2005). Oncogene-induced senescence as an initial barrier in lymphoma development. Nature 436, 660-665.
Campisi,J. (2011). Cellular senescence: putting the paradoxes in perspective. Curr. Opin. Genet. Dev. 21, 107-112.
Chen,Z., Trotman,L.C., Shaffer,D., Lin,H.K., Dotan,Z.A., Niki,M., Koutcher,J.A., Scher,H.I., Ludwig,T., Gerald,W., Cordon-Cardo,C., and Paolo Pandolfi,P. (2005). Crucial role of p53-dependent cellular senescence in suppression of Pten-deficient tumorigenesis. Nature 436, 725-730.
Collado,M., Gil,J., Efeyan,A., Guerra,C., Schuhmacher,A.J., Barradas,M., Benguria,A., Zaballos,A., Flores,J.M., Barbacid,M., Beach,D., and Serrano,M. (2005). Tumour biology: Senescence in premalignant tumours. Nature 436, 642.
Engelholm,L.H., Ingvarsen,S., Jurgensen,H.J., Hillig,T., Madsen,D.H., Nielsen,B.S., and Behrendt,N. (2009). The collagen receptor uPARAP/Endo180. Front Biosci. 14, 2103-2114.
Ewald,J.A., Desotelle,J.A., Wilding,G., and Jarrard,D.F. (2010). Therapy-induced senescence in cancer. J. Natl. Cancer Inst. 102, 1536-1546.
Hanahan,D. and Weinberg,R.A. (2000). The hallmarks of cancer. Cell 100, 57-70.
Lambeau,G. and Lazdunski,M. (1999). Receptors for a growing family of secreted phospholipases A2. Trends Pharmacol. Sci. 20, 162-170.
Murakami,M., Taketomi,Y., Girard,C., Yamamoto,K., and Lambeau,G. (2010). Emerging roles of secreted phospholipase A2 enzymes: Lessons from transgenic and knockout mice. Biochimie 92, 561-582.
Rodier,F. and Campisi,J. (2011). Four faces of cellular senescence. J. Cell Biol. 192, 547-556.
Shrimpton,R.E., Butler,M., Morel,A.S., Eren,E., Hue,S.S., and Ritter,M.A. (2009). CD205 (DEC-205): a recognition receptor for apoptotic and necrotic self. Mol. Immunol. 46, 1229-1239.
Taylor,P.R., Gordon,S., and Martinez-Pomares,L. (2005). The mannose receptor: linking homeostasis and immunity through sugar recognition. Trends Immunol. 26, 104-110.

## Claims

1. Composition for use in a method for the treatment of cancer, said composition comprising a polynucleotide selected in the group consisting of:
- The polynucleotide of SEQ ID NO. 1 or a variant thereof having at least 95% identity with the sequence of SEQ ID No. 1;
- A polynucleotide encoding the human PLA2R1 (Phospholipase A2 receptor 1) polypeptide of SEQ ID NO. 2 or encoding a variant thereof having at least 95% identity with the sequence of SEQ ID No. 2;
- A polynucleotide from position 1 to position 4191 of SEQ ID NO.1 encoding the extracellular domain of human PLA2R1 or a variant thereof having at least 95% identity with the polynucleotide from position 1 to position 4191 of SEQ ID NO.1;
- A fragment of the polynucleotide from position 1 to position 4191 of SEQ ID NO.1 encoding a fragment of the extracellular domain of PLA2R1 or a variant thereof having at least 95% identity with a fragment of the polynucleotide from position 1 to position 4191 of SEQ ID NO.1, said fragment encoding at least the Cys-rich domain, the fibronectin type II domain and a C-type lectin domain of human PLA2R1; or
- A polynucleotide encoding a polypeptide of SEQ ID NO. 4, 6 or 8

2. Composition for use in a method for the treatment of cancer according to claim 1 wherein the fragment of the polynucleotide from position 1 to position 4191 of SEQ ID NO.1 is selected in the group consisting of the polynucleotides of SEQ ID NO. 3, SEQ ID NO. 5, SEQ ID NO. 7 and variants thereof having at least 95% identity with the polynucleotide of SEQ ID NO. 3, SEQ ID NO.5 or SEQ ID NO.7.

3. Composition for use in a method for the treatment of cancer according to anyone of claims 1-2 wherein said polynucleotide is inserted in a viral vector.

4. Composition for use in a method for the treatment of cancer according to anyone of claims 1-2 wherein said polynucleotide is incorporated in a delivery vehicle.

5. Composition for use in a method for the treatment of cancer, said composition comprising a polypeptide selected in the group consisting of:
- The Human PLA2R1 polypeptide depicted in SEQ ID NO. 2 or a variant thereof having at least 95% identity with the polypeptide of SEQ ID No. 2;
- The extracellular domain of human PLA2R1 polypeptide from position 21 to position 1397 of SEQ ID NO.2 or a variant thereof having at least 95 % identity with the human PLA2R1 polypeptide from position 21 to position 1397 of SEQ ID NO.2; or
- A fragment of the extracellular domain of human PLA2R1 polypeptide from position 21 to position 1397 of SEQ ID NO.2 or a variant thereof having at least 95 % identity with a fragment the human PLA2R1 polypeptide from position 21 to position 1397 of SEQ ID NO.2, said fragment comprising at least the Cys-rich domain, the fibronectin type II domain and a C-type lectin domain of human PLA2R1

6. Composition for use in a method for the treatment of cancer according to claim 5 wherein the fragment of the extracellular domain of human PLA2R1 polypeptide from position 21 to position 1397 of SEQ ID NO.2 is selected in the group consisting of the polypeptide from position 21 to position 1322 of SEQ ID NO. 4, the polypeptide from position 21 to position 1098 of SEQ ID NO. 6, the polypeptide from position 21 to position 645 of SEQ ID NO. 8 and variants thereof having at least 95% identity with the polypeptide from position 21 to position 1322 of SEQ ID NO. 4, at least 95% identity with the polypeptide from position 21 to position 1098 of SEQ ID NO. 6 or at least 95% identity with the polypeptide from position 21 to position 645 of SEQ ID NO. 8.

7. Composition for use in a method for the treatment of cancer according to anyone of claims 1-6, wherein said cancer is selected in the group consisting of breast cancer, pancreatic cancer, kidney cancer, colorectal cancer and melanoma.

8. Polypeptide encoding a soluble extracellular fragment of human PLA2R1 consisting of the polypeptide of SEQ ID NOs. 4, 6, 8.

9. Polypeptide according to claim 8 for use in a method for the treatment of cancer.

10. Polynucleotide encoding a polypeptide according to claim 8.

11. Polynucleotide according to claim 10 for use in a method for the treatment of cancer.

12. Method for determining the prognosis of a cancer in a patient comprising the following steps:
a) Measuring the level of expression of the PLA2R1 gene in cancer cells from a tumour sample previously taken from said patient,
b) Classifying the cancer as having a poor prognosis if the PLA2R1 gene is under-expressed in said cancer cells compared to a control sample.

13. Method according to claim 12, wherein the cancer is a breast cancer or a kidney cancer.

## Patentansprüche

1. Zusammensetzung zur Verwendung in einem Verfahren zur Behandlung einer Krebserkrankung, wobei die genannte Zusammensetzung ein Polynukleotid umfasst, das ausgewählt ist aus der Gruppe, bestehend aus:
- dem Polynukleotid der SEQ ID NR. 1 oder einer Variante davon, die wenigstens zu 95 % mit der Sequenz der SEQ ID NR. 1 identisch ist;
- einem Polynukleotid, das das menschliche PLA2R1 (Phospholipase A2 Rezeptor 1) Polypeptid der SEQ ID NR. 2 kodiert oder eine Variante davon kodiert, die wenigstens zu 95 % mit der Sequenz der SEQ ID NR. 2 identisch ist;
- einem Polynukleotid von Position 1 bis Position 4191 der SEQ ID NR. 1, das den extrazellulären Bereich des menschlichen PLA2R1 oder eine Variante davon kodiert, die wenigstens zu 95 % mit dem Polynukleotid von Position 1 bis Position 4191 der SEQ ID NR. 1 identisch ist;
- einem Fragment des Polynukleotids von Position 1 bis Position 4191 der SEQ ID NR. 1, die ein Fragment des extrazellulären Bereichs von PLA2R1 oder eine Variante davon kodiert, die wenigstens zu 95 % mit einem Fragment des Polynukleotids von Position 1 bis Position 4191 der SEQ ID NR. 1 identisch ist, wobei das genannte Fragment wenigstens den Cys-reichen Bereich, den Fibronektin Typ II-Bereich und einen Lektinbereich vom C-Typ des menschlichen PLA2R1 kodiert;
oder
- ein Polynukleotid, das ein Polypeptid der SEQ ID NR. 4, 6, oder 8 kodiert.

2. Zusammensetzung zur Verwendung in einem Verfahren zur Behandlung einer Krebserkrankung gemäß Anspruch 1, wobei das Fragment des Polynukleotids von Position 1 bis Position 4191 der SEQ ID NR. 1 ausgewählt ist aus der Gruppe bestehend aus den Polynukleotiden der SEQ ID NR. 3, SEQ ID NR. 5, SEQ ID NR. 7 und Varianten davon, die wenigstens zu 95 % mit dem Polynukleotid der SEQ ID NR. 3, SEQ ID NR. 5 oder SEQ ID NR. 7 identisch ist.

3. Zusammensetzung zur Verwendung in einem Verfahren zur Behandlung einer Krebserkrankung gemäß irgendeinem der Ansprüche 1 bis 2, wobei das genannte Polynukleotid in einen viralen Vektor eingefügt ist.

4. Zusammensetzung zur Verwendung in einem Verfahren zur Behandlung einer Krebserkrankung gemäß irgendeinem der Ansprüche 1 bis 2, wobei das genannte Polynukleotid in einen Anwendungsträger eingebettet ist.

5. Zusammensetzung zur Verwendung in einem Verfahren zur Behandlung einer Krebserkrankung, wobei die genannte Zusammensetzung ein Polypeptid umfasst, das ausgewählt ist aus der Gruppe, bestehend aus:
- dem menschlichen PLA2R1 Polypeptid, das in der SEQ ID NR. 2 oder einer Variante davon, die wenigstens zu 95 % mit dem Polypeptid der SEQ ID NR. 2 identisch ist, dargestellt ist,
- dem extrazellulären Bereich von menschlichem PLA2R1 Polypeptid von Position 1 bis Position 1397 der SEQ ID NR. 2 oder einer Variante davon, die wenigstens zu 95 % mit dem menschlichen PLA2R1 Polypeptid von Position 21 bis Position 1397 der SEQ ID NR. 2 identisch ist;
oder
- Fragment des extrazellulären Bereichs von menschlichem PLA2R1 Polypeptid von Position 21 bis Position 1397 der SEQ ID NR. 2 oder einer Variante davon, die wenigstens zu 95 % mit einem Fragment des menschlichen PLA2R1 Polypeptids von Position 21 bis Position 1397 der SEQ ID NR. 3 identisch ist, wobei das genannte Fragment wenigstens den Cys-reichen Bereich, den Fibronektin Typ II-Bereich und einen Lektinbereich vom C-Typ des menschlichen PLA2R1 umfasst.

6. Zusammensetzung zur Verwendung in einem Verfahren zur Behandlung einer Krebserkrankung gemäß Anspruch 5, wobei das Fragment des extrazellulären Bereichs von menschlichem PLA2R1 Polypeptid von Position 21 bis Position 1397 der SEQ ID NR. 2 aus der Gruppe ausgewählt ist, bestehend aus dem Polypeptid von Position 21 bis Position 1322 der SEQ ID NR. 4, dem Polypeptid von Position 21 bis Position 1098 der SEQ ID NR. 6, dem Polypeptid von Position 21 bis Position 645 der SEQ ID NR. 8 und Varianten davon, die wenigstens zu 95 % mit dem Polypeptid von Position 21 bis Position 1322 der SEQ ID NR. 4 identisch sind, wenigstens zu 95 % mit dem Polypeptid von Position 21 bis Position 1098 der SEQ ID NR. 6 identisch sind oder wenigstens zu 95 % mit dem Polypeptid von Position 21 bis Position 645 der SEQ ID NR. 8 identisch sind.

7. Zusammensetzung zur Verwendung in einem Verfahren zur Behandlung einer Krebserkrankung gemäß irgendeinem der Ansprüche 1 bis 6, wobei die genannte Krebserkrankung ausgewählt ist aus der Gruppe bestehend aus Brustkrebs, Bauchspeicheldrüsenkrebs, Nierenkrebs, Darmkrebs und Melanomen.

8. Polypeptid, das ein lösliches extrazelluläres Fragment von menschlichem PLA2R1 kodiert, das aus dem Polypeptid der SEQ ID NR. 4, 6, 8 gebildet ist.

9. Polypeptid gemäß Anspruch 8 zur Verwendung in einem Verfahren zur Behandlung einer Krebserkrankung.

10. Polynukleotid, das ein Polypeptid gemäß Anspruch 8 kodiert.

11. Polynukleotid gemäß Anspruch 10 zur Verwendung in einem Verfahren zu Behandlung einer Krebserkrankung.

12. Verfahren zur Bestimmung der Prognose eines Krebses bei einem Patienten, umfassend die folgenden Schritte:
a) Messen des Expressionsniveaus des PLA2R1-Gens in Krebszellen aus einer zuvor dem genannten Patienten entnommenen Tumorprobe,
b) Einstufung der Krebserkrankung mit einer schlechten Prognose, wenn das PLA2R1 Gen im Vergleich zu einer Kontrollprobe in den genannten Krebszellen unterexprimiert ist.

13. Verfahren gemäß Anspruch 12, wobei die Krebserkrankung ein Brustkrebs oder ein Nierenkrebs ist.

## Revendications

1. Composition destinée à être utilisée dans un procédé pour traiter le cancer, ladite composition comprenant un polynucléotide choisi dans le groupe constitué :
- du polynucléotide de SEQ ID NO. 1 ou d'un variant de celui-ci ayant une identité d'au moins 95 % avec la séquence de SEQ ID NO. 1 ;
- d'un polynucléotide codant pour le polypeptide PLA2R1 (récepteur 1 de la phospholipase A2) humain de SEQ ID NO. 2 ou codant pour un variant de celui-ci ayant une identité d'au moins 95 % avec la séquence de SEQ ID NO. 2 ;
- d'un polynucléotide situé de la position 1 à la position 4191 de SEQ ID NO. 1 codant pour le domaine extracellulaire du PLA2R1 humain ou d'un variant de celui-ci ayant une identité d'au moins 95 % avec le polynucléotide situé de la position 1 à la position 4191 de SEQ ID NO. 1 ;
- d'un fragment du polynucléotide situé de la position 1 à la position 4191 de SEQ ID NO. 1 codant pour un fragment du domaine extracellulaire du PLA2R1 ou d'un variant de celui-ci ayant une identité d'au moins 95 % avec un fragment du polynucléotide situé de la position 1 à la position 4191 de SEQ ID NO. 1, ledit fragment codant pour au moins le domaine riche en Cys, le domaine fibronectine de type II et un domaine lectine de type C du PLA2R1 humain ; ou
- d'un polynucléotide codant pour un polypeptide de SEQ ID NO. 4, 6 ou 8.

2. Composition destinée à être utilisée dans un procédé pour traiter le cancer selon la revendication 1, dans laquelle le fragment du polynucléotide situé de la position 1 à la position 4191 de SEQ ID NO. 1 est choisi dans le groupe constitué des polynucléotides de SEQ ID NO. 3, SEQ ID NO. 5, SEQ ID NO. 7 et des variants de ceux-ci ayant une identité d'au moins 95 % avec le polynucléotide de SEQ ID NO. 3, SEQ ID NO. 5 ou SEQ ID NO. 7.

3. Composition destinée à être utilisée dans un procédé pour traiter le cancer selon l'une quelconque des revendications 1 ou 2, dans laquelle ledit polynucléotide est inséré dans un vecteur viral.

4. Composition destinée à être utilisée dans un procédé pour traiter le cancer selon l'une quelconque des revendications 1 ou 2, dans laquelle ledit polynucléotide est incorporé dans un véhicule d'administration.

5. Composition destinée à être utilisée dans un procédé pour traiter le cancer, ladite composition comprenant un polypeptide choisi dans le groupe constitué :
- du polypeptide PLA2R1 humain illustré dans SEQ ID NO. 2 ou d'un variant de celui-ci ayant une identité d'au moins 95 % avec le polypeptide de SEQ ID NO. 2 ;
- du domaine extracellulaire du polypeptide PLA2R1 humain situé de la position 21 à la position 1397 de SEQ ID NO. 2 ou d'un variant de celui-ci ayant une identité d'au moins 95 % avec le polypeptide PLA2R1 humain situé de la position 21 à la position 1397 de SEQ ID NO. 2 ; ou
- d'un fragment du domaine extracellulaire du polypeptide PLA2R1 humain situé de la position 21 à la position 1397 de SEQ ID NO. 2 ou d'un variant de celui-ci ayant une identité d'au moins 95 % avec un fragment du polypeptide PLA2R1 humain situé de la position 21 à la position 1397 de SEQ ID NO. 2, ledit fragment comprenant au moins le domaine riche en Cys, le domaine fibronectine de type II et un domaine lectine de type C du PLA2R1 humain.

6. Composition destinée à être utilisée dans un procédé pour traiter le cancer selon la revendication 5, dans laquelle le fragment du domaine extracellulaire du polypeptide PLA2R1 humain situé de la position 21 à la position 1397 de SEQ ID NO. 2 est choisi dans le groupe constitué du polypeptide situé de la position 21 à la position 1322 de SEQ ID NO. 4, du polypeptide situé de la position 21 à la position 1098 de SEQ ID NO. 6, du polypeptide situé de la position 21 à la position 645 de SEQ ID NO. 8 et des variants de ceux-ci ayant une identité d'au moins 95 % avec le polypeptide situé de la position 21 à la position 1322 de SEQ ID NO. 4, d'au moins 95 % avec le polypeptide situé de la position 21 à la position 1098 de SEQ ID NO. 6 ou d'au moins 95 % avec le polypeptide situé de la position 21 à la position 645 de SEQ ID NO. 8.

7. Composition destinée à être utilisée dans un procédé pour traiter le cancer selon l'une quelconque des revendications 1 à 6, dans laquelle ledit cancer est choisi dans le groupe constitué du cancer du sein, du cancer du pancréas, du cancer du rein, du cancer colorectal et du mélanome.

8. Polypeptide codant pour un fragment extracellulaire soluble de PLA2R1 humain constitué du polypeptide de SEQ ID NO. 4, 6 ou 8.

9. Polypeptide selon la revendication 8 destiné à être utilisé dans un procédé pour traiter le cancer.

10. Polynucléotide codant pour un polypeptide selon la revendication 8.

11. Polynucléotide selon la revendication 10 destiné à être utilisé dans un procédé pour traiter le cancer.

12. Procédé pour déterminer le pronostic d'un cancer chez un patient comprenant les étapes suivantes :
a) mesurer le niveau d'expression du gène PLA2R1 dans des cellules cancéreuses issues d'un échantillon tumoral prélevé au préalable sur ledit patient,
b) classer le cancer comme étant de mauvais pronostic si le gène PLA2R1 est sous-exprimé dans lesdites cellules cancéreuses par rapport à un échantillon témoin.

13. Procédé selon la revendication 12, dans lequel le cancer est le cancer du sein ou le cancer du rein.
